# EUROPEAN PATENT SPECIFICATION

(11) **EP 1 856 256 B1**
(45) Date of publication and mention of the grant of the patent: **15.07.2009**
(21) Application number: 06733839.2
(22) Date of filing: 20.01.2006
(51) Int. Cl.: C12N 15/10, C12Q 1/68

(54) **FREE REACTANT USE IN NUCLEIC ACID-TEMPLATED SYNTHESIS**
VERWENDUNG FREIER REAKTANTEN BEI DER NUKLEINSÄURETEMPLATGESTEUERTEN SYNTHESE
UTILISATION DE RÉACTIF LIBRE DANS LA SYNTHÈSE FAISANT APPEL À UNE MATRICE D'ACIDE NUCLÉIQUE

(30) Priority: 21.01.2005 US 646584 P
(43) Date of publication of application: 21.11.2007
(73) Proprietor: President and Fellows of Harvard College, Cambridge, MA 02138 (US)
(72) Inventor: LIU, David, Lexington, Massachusetts 02420 (US); SAKURAI, Kaori, Tokyo 167-0043 (US)
(74) Representative: White, Martin Paul
(86) International application number: PCT/US2006/002420
(87) International publication number: WO 2006/079061

(56) References cited:
- WO-A-00/23458
- WO-A-93/20242
- WO-A-02/103008
- WO-A-20/04013070
- WO-A-20/04016767
- LI XIAOYU ET AL: "DNA-templated organic synthesis: nature's strategy for controlling chemical reactivity applied to synthetic molecules." ANGEWANDTE CHEMIE (INTERNATIONAL ED. IN ENGLISH) 20 SEP 2004, vol. 43, no. 37, 20 September 2004 (2004-09-20), pages 4848-4870, XP002392408 ISSN: 0570-0833 cited in the application

### SEQUENCE LISTING

<110> President and Fellows of Harvard college
   Liu, David R.
   Sakurai, Kaori
<120> Free Reactant Use in Nucleic Acid-Templated Synthesis
<130> LS1-003PC
<150> US 60/646,584
   <151> 2005-01-21
<160> 13
<170> PatentIn version 3.3
<210> 1
   <211> 30
   <212> DNA
   <213> Artificial
<220>
   <223> 30-mer oligonucleotide Template (for characterization by PAGE)
<400> 1 30
   ggtacgaatt gcactcggga aatccacctt 30
<210> 2
   <211> 10
   <212> DNA
   <213> Artificial
<220>
   <223> 10-mer oligonucleotide Linked to Phosphine
<400> 2 10
   aattcgtacc 10
<210> 3
   <211> 10
   <212> DNA
   <213> Artificial
<220>
   <223> 10-mer oligonucleotide with three-base mismatch
<400> 3
   aatacatccc 10
<210> 4
   <211> 20
   <212> DNA
   <213> Artificial
<220>
   <223> 20-mer oligonucleotide capture Reagent
<400> 4
   tcccgagtgc aattcgtacc 20
<210> 5
   <211> 10
   <212> DNA
   <213> Artificial
<220>
   <223> 10-mer oligonucleotide Template (for characterization by Mass Spetroscopy)
<400> 5
   ggtacgaatt 10
<210> 6
   <211> 14
   <212> DNA
   <213> Artificial
<220>
   <223> Template 13 oligonucleotide
<400> 6
   ttgtgcaacg tcat 14
<210> 7
   <211> 23
   <212> DNA
   <213> Artificial
<220>
   <223> Template 14 oligonucleotide
<400> 7
   ttcctagtcg tcatgtagta gta 23
<210> 8
   <211> 20
   <212> DNA
   <213> Artificial
<220>
   <223> Template 15 oligonucleotide
<400> 8
   tttaagcccg tcatgtagta 20
<210> 9
   <211> 17
   <212> DNA
   <213> Artificial
<220>
   <223> Template 16 oligonucleotide
<400> 9
   ttagcttgcg tcatgta 17
<210> 10
   <211> 10
   <212> DNA
   <213> Artificial
<220>
   <223> Reactant 17 oligonucleotide
<400> 10
   cgttgcacaa 10
<210> 11
   <211> 10
   <212> DNA
   <213> Artificial
<220>
   <223> Reactant 18 oligonucleotide
<400> 11
   cgactaggaa 10
<210> 12
   <211> 10
   <212> DNA
   <213> Artificial
<220>
   <223> Reactant 19 oligonucleotide
<400> 12
   cgggcttaaa 10
<210> 13
   <211> 10
   <212> DNA
   <213> Artificial
<220>
   <223> Reactant 20 oligonucleotide
<400> 13
   cgcaagctaa 10

## Description

### FIELD OF THE INVENTION

The invention relates generally to methods for performing nucleic acid-templated synthesis. More particularly, the invention relates to methods for performing nucleic acid-templated synthesis to produce reaction intermediates, which can then be chemically transformed into reaction products using free reactants that react with the reaction intermediates to produce the reaction products.

### BACKGROUND OF THE INVENTION

Nucleic acid-templated organic synthesis enables modes of controlling reactivity that are not possible in a conventional synthesis format and allows synthetic molecules to be manipulated using translation, selection, and amplification methods previously available only to biological macromolecules (Gartner et al. (2001) J. AM. CHEM. Soc. 123: 6961-3; Gartner et al. (2002) ANGEW. CHEM., INT. ED. ENGL. 123: 61796-1800; Gartner et al. (2002) J. AM. CHEM. SOC. 124: 10304-6; Calderone et al. (2002) ANGEW. CHEM., INT. ED. ENGL. 41: 4104-8; Gartner et al. (2003) ANGEW. CHEM., INT. ED. ENGL. 42: 1370-5; Li et al. (2004) J. AM. CHEM. SOC. 124: 5090-2; Kanan et al. (2004) NATURE 431: 545-9; Gartner et al. (2004) SCIENCE 305: 1601-5; Li et al. (2004) ANGEW. CHEM. INT. ED. 43: 4848-70; Brenner et al. (1992) PROC. NATL. ACAD. SCI. USA 89: 5181; Doyon et al. (2003) J. AM. CHEM. SOC. 125: 12372-3; Halpin et al. (2004) PLoS BIOL. 2: e174).
WO 2004/013070 is entitled "Multi-Step Synthesis of Templated Molecules". It describes a method for the manufacture of a library of complexes. The complexes comprise templated molecules attached to the template which directed the synthesis thereof. The templated molecules are produced in a step-by-step fashion which provides for a high local concentration of reactive groups involved in the formation of connections between the individual components of the template molecule.
WO 2004/016767 is entitled "Evolving New Molecular Function". It explains that nature evolves biological molecules such as proteins through iterated rounds of diversification, selection, and amplification. It provides a variety-of template architectures for performing nucleic acid-templated synthesis, methods for increasing the selectivity of nucleic acid-templated reactions, methods for performing stereoselective nucleic acid-templated reactions, methods of selecting for reaction products resulting from nucleic acid-templated synthesis, and methods of identifying new chemical reactions based on nucleic acid-templated synthesis.
WO 2002/103008 is entitled "templated molecules and methods for using such molecules". It is said to relate to templated molecules and complexes comprising such molecules linked to the template that has directed the template-directed synthesis of the templated molecule. The invention allows the generation of libraries which can be screened for e.g. therapeutic activity.
An article by Li and Liu (Angewandte Chemie, International Edition, 43, 4848-4870 [2004]) describes DNA templated organic synthesis, referred to therein as "DTS". Here it is explained that DTS is emerging as a surprisingly general way to control the reactivity of synthetic molecules by using nature' effective-molarity-based approach. It is also pointed out that recent developments have expanded the scope and capabilities of DTS from its origins as a model of prebiotic nucleic acid replication to its current ability to translate DNA sequences into complex small-molecule and polymer products of multistep organic synthesis. Early applications of DTS are said to include nucleic acid sensing, small-molecule discovery, and reaction discovery with the help of translation, selection, and amplification methods previously available only to biological molecules.
The structures that can be accessed through nucleic acid-templated synthesis, in particular, DNA-templated organic synthesis, or DTS, have been limited predominantly to products of coupling reactions between two nucleic acid-linked reactants. In some cases, however, reactants are difficult or impossible to tether to an oligonucleotide. The development of strategies that enable non-oligonucleotide linked small-molecule reagents to react in a sequence-programmed or sequence-recorded manner, therefore, would significantly expand the synthetic capabilities of nucleic acid-templated synthesis.

### SUMMARY OF THE INVENTION

The present invention provides methods for expanding the scope of nucleic acid-templated organic syntheses by addressing the need for reagents to be tethered to oligonucleotides. When the linkage of reagents to a nucleic acid, for example, DNA, is not possible or convenient, these transformations allow such reagents to nevertheless contribute to small molecule syntheses while preserving the correspondence between nucleic acid sequence and the structure of the product. In addition, by decoupling the nucleic acid-templated step from the coupling reaction, this approach allows bond formation to take place under conditions that do not necessarily support nucleic acid hybridization.

In one aspect, the invention provides a method of synthesizing a reaction product as set forth in claim 1.

In another aspect, the invention provides a method of synthesizing a reaction product via nucleic acid-templated synthesis as set forth in claim 6.

To the extent that multiple different first reactive units (and optionally second reactive units) are present in an initial reaction mixture, it is possible that, under certain reaction conditions, multiple different reaction intermediates may be created. Accordingly, it may be advantageous for the free reactant to be selectively reactive with just one specific type of reaction intermediate in the mixture. Alternatively, it may be advantageous for the free reactant to be selectively reactive with a group or sub-group of reaction intermediates, where the reaction intermediates have a functional group with a particular chemical functionality. For example, the free reactant may be selectively reactive with reactive intermediates containing a free amine as compared to other reactive intermediates lacking a free amine.

Furthermore, by knowing the codon and/or anticodon sequences it is possible to determine which second reactive unit reacted with the first reactive unit to produce the reactive intermediate and/or the reaction product. Furthermore, if the first oligonucleotide provides a sequence identifier for the first reactive unit attached to the first oligonucleotide, it is possible to determine what first reaction unit reacted with the second reactive unit to produce the reaction intermediate and/or the reaction product. Based upon the reaction conditions, the reactants present in a reaction mixture containing reaction intermediates, and information concerning when certain free reactants are added to the mixture containing reaction intermediates, it can be possible to determine what free reactant reacted with a reaction intermediate to create a specific reaction product. This information can be used to identify the reaction product and the reaction pathway by which it was made.

The foregoing aspects and features of the invention may be further understood by reference to the following drawings, detailed description, examples, and claims.

### DEFINITIONS

The terms, "codon" and "anti-codon" as used herein, refer to complementary oligonucleotide sequences in a template and in a transfer unit, respectively, that permit the transfer unit to anneal to the template during nucleic acid-templated synthesis.

The term, "free reactant" as used herein refers to a chemical reagent or chemical moiety that is not linked to an oligonucleotide that can participate in nucleic acid-templated synthesis. In comparison, the first and second reactive units and the transfer units are attached to oligonucleotides that can participate in nucleic acid-templated synthesis.

The terms, "oligonucleotide" or "nucleic acid" as used herein refer to a polymer of nucleotides. The polymer may include, without limitation, natural nucleosides (*i.e.*, adenosine, thymidine, guanosine, cytidine, uridine, deoxyadenosine, deoxythymidine, deoxyguanosine, and deoxycytidine), nucleoside analogs (*e.g.*, 2-aminoadenosine, 2-thiothymidine, inosine, pyrrolopyrimidine, 3-methyl adenosine, 5-methylcytidine, C5-bromouridine, C5-fluorouridine C5-iodouridine, C5-propynyl-uridine, C5-propynyl-cytidine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (*e.g.*, methylated bases), intercalated bases, modified sugars (*e.g.*, 2'-fluororibose, ribose, 2'-deoxyribose, arabinose, and hexose), or modified phosphate groups (*e.g.*, phosphorothioates and 5' -N-phosphoramidite linkages). Nucleic acids and oligonucleotides may also include other polymers of bases having a modified backbone, such as a locked nucleic acid (LNA), a peptide nucleic acid (PNA), a threose nucleic acid (TNA) and any other polymers capable of serving as a template for an amplification reaction using an amplification technique, for example, a polymerase chain reaction, a ligase chain reaction, or non-enzymatic template-directed replication.

The term, "reactive unit" as used herein, refers to a chemical reagent or chemical moiety (including, for example, but not limited to, a building block, monomer, monomer unit, small molecule scaffold, or other reactant useful in nucleic acid-templated chemical synthesis) that can participate in a chemical reaction with another chemical reagent or chemical moiety to produce a reaction intermediate and/or a reaction product.

The term, "reaction intermediate" as used herein, refers to a chemical reagent or a chemical moiety that can be chemically transformed into a different reagent or chemical moiety with a free reactant.

The term, "small molecule" as used herein, refers to an organic compound either synthesized in the laboratory or found in nature having a molecular weight less than 10,000 grams per mole, optionally less than 5,000 grams per mole, and optionally less than 2,000 grams per mole.

The term, "small molecule scaffold" as used herein, refers to a chemical compound having at least one site or chemical moiety suitable for functionalization. The small molecule scaffold or molecular scaffold may have two, three, four, five or more sites or chemical moieties suitable for functionalization. These functionalization sites may be protected or masked as would be appreciated by one of skill in this art. The sites may also be found on an underlying ring structure or backbone. The small molecule scaffolds are not nucleic acids, nucleotides, or nucleotide analogs.

The term, "transfer unit" as used herein, refers to a molecule comprising an oligonucleotide having an anti-codon sequence attached to a reactive unit including, for example, but not limited to, a building block, monomer, monomer unit, small molecule scaffold, or other reactant useful in nucleic acid-templated chemical synthesis.

The term, "template" as used herein, refers to a molecule comprising an oligonucleotide having at least one codon sequence suitable for a nucleic acid-templated chemical synthesis. The template optionally may comprise (i) a plurality of codon sequences, (ii) an amplification means, for example, a PCR primer binding site or a sequence complementary thereto, (iii) a reactive unit associated therewith, (iv) a combination of (i) and (ii), (v) a combination of (i) and (iii), (vi) a combination of (ii) and (iii), or a combination of (i), (ii) and (iii).

Throughout the description, where compositions are described as having, including, or comprising specific components, or where methods are described as having, including, or comprising specific process steps, it is contemplated that compositions also consist essentially of, or consist of, the recited components, and that the methods of the present invention also consist essentially of, or consist of, the recited method steps. Further, it should be understood that the order of steps or order for performing certain actions are immaterial so long as the invention remains operable. Moreover, unless specified to the contrary, two or more steps or actions may be conducted simultaneously.

### DESCRIPTION OF THE DRAWINGS

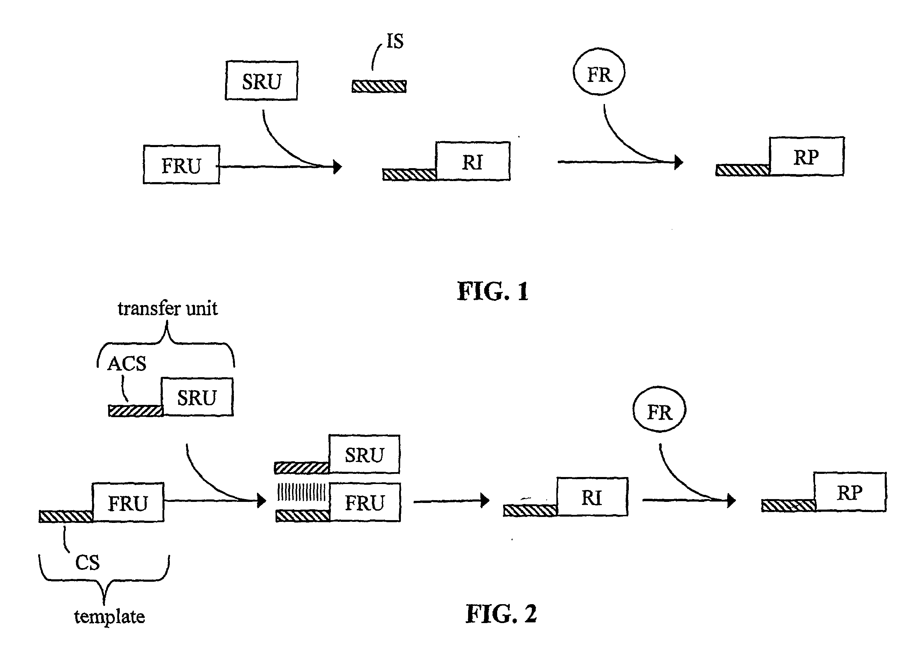

**FIG. 1** is a schematic illustration of one aspect of the present invention in which a first reactive unit (FRU) and a second reactive unit (SRU) react to form a reaction intermediate (RI). The reaction intermediate (RI) is attached to an identifying sequence (IS). The RI-IS complex is combined with a free reactant (FR), which is selectively reactive with the RI to yield a reaction product (RP) linked to the IS.

**FIG. 2** is a schematic illustration of an aspect of the present invention, in which prior to the formation of the reaction intermediate (RI) the first reactive unit (FRU) and the second reactive unit (SRU) are linked to a codon sequence (CS) and a complementary anti-codon sequence (ACS), respectively. The CS and the ACS anneal to one another to permit the FRU and SRU to react with one another to produce the RI. The CS remains linked to the RI. The CS remains attached to the reaction product (RP) when the RI has been reacted with the free reactant (FR).

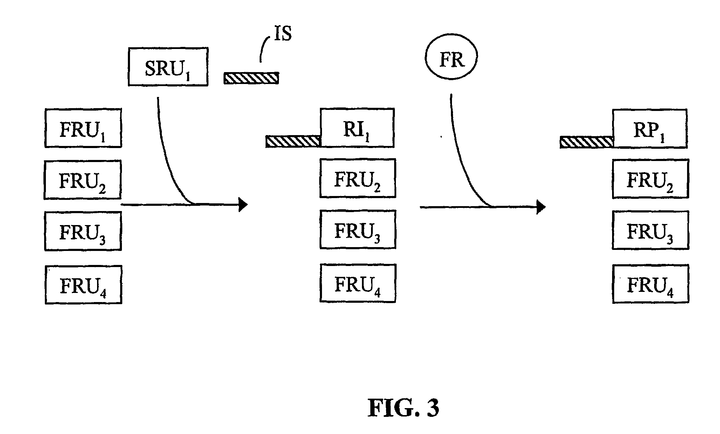

**FIG. 3** is a schematic illustration of an embodiment of the scheme shown in **FIG. 1****,** in which, in a mixture of a population of first reactive units (FRU₁-FRU₄), at least one of the first reactive units (FRU₁) reacts with a second reactive unit (SRU₁) to form a reaction intermediate (RI₁) which coexists with the population of first reactive units (FRU₂-FRU₄). The reaction intermediate (RI₁) is linked to an identifying sequence (IS). The reaction intermediate (RI₁) linked to the IS and coexisting with the population of first reactive units (FRU₂-FRU₄) then is permitted to react with a free reactant (FR), which is selectively reactive with RI₁, to yield a reaction product (RP₁) linked to the IS.

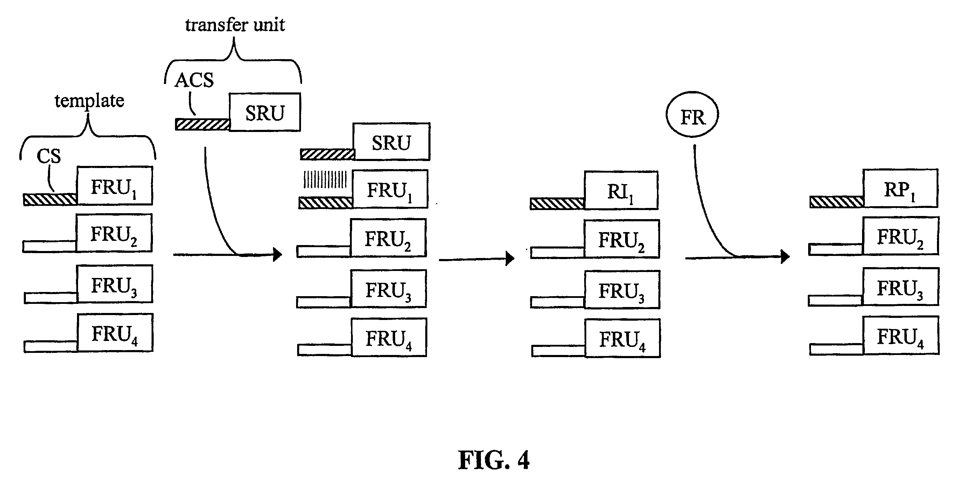

**FIG. 4** is a schematic illustration of an embodiment of the scheme shown in **FIG. 2****,** in which prior to the formation of the reaction intermediate (RI₁) the first reactive units (FRU₁-FRU₄) and the second reactive unit (SRU) are linked to codon sequences (CS) and a complementary anti-codon sequence (ACS), respectively. The CS remains linked to RI₁. The CS remains attached to the reaction product (RP₁) when RI₁ has been reacted with the free reactant (FR) to produce RP₁.

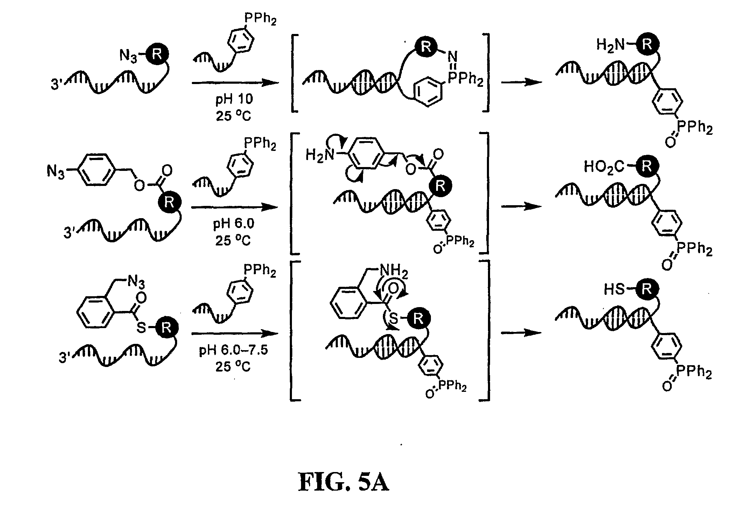

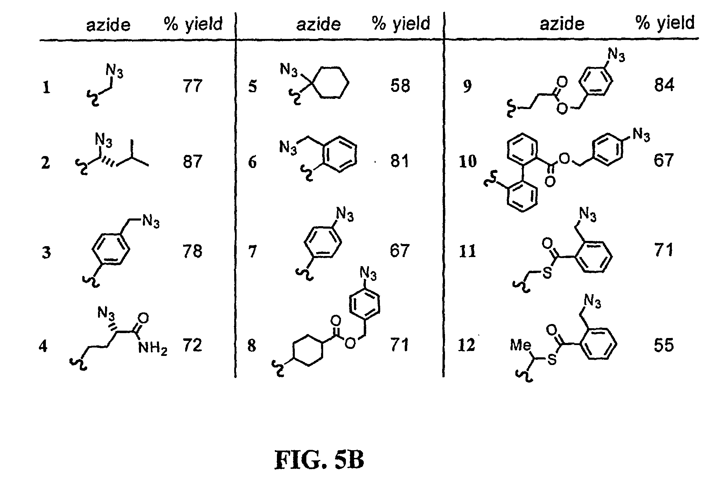

**FIG. 5A** depicts DNA-templated transformation of azides into primary amines (top scheme), carboxylic acids (middle scheme), and thiols (bottom scheme). **FIG. 5B** depicts exemplary DNA-templated reactions using substrate azides **1-12,** in which the listed yields represent lower limits.

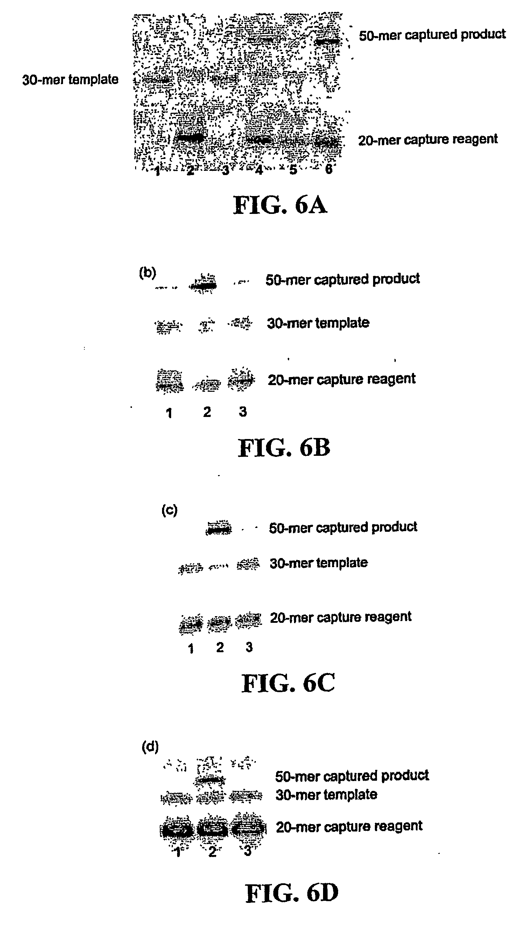

**FIG. 6A** is a representation of a denaturing polyacrylamide gel electrophoresis (PAGE) gel following DNA-templated azide-to-amine transformation of azide **3** from **FIG. 5B****.** Lane 1 contains azide-linked 30-mer template. Lane 2 contains azide-linked 30-mer template + carboxylic acid-linked 20-mer capture reagent + 30 mM EDC + 15 mM sulfo-NHS (showing no product formation). Lane 3 contains azide-linked 30-mer template + phosphine-linked 10-mer reagent (10-mer not visible). Lane 4 contains azide-linked 30-mer template + phosphine-linked 10-mer reagent + carboxylic acid-linked 20-mer capture reagent + 30 mM EDC + 15 mM sulfo-NHS, the 50-mer secondary product arising from azide-to-amine transformation following by DNA-templated amine acylation is visible. Lane 5 contains azide-linked 30-mer template + phosphine-linked 10-mer reagent containing a mismatched sequence + carboxylic acid-linked 20-mer capture reagent + 30 mM EDC + 15 mM sulfo-NHS. Lane 6 contains azide-linked 30-mer template + 5 mM TCEP-HCl + carboxylic acid-linked 20-mer capture reagent + 30 mM EDC + 15 mM sulfo-NHS (positive control in which the azide is reduced *in situ* by TCEP). It appears that incomplete denaturing of the duplex between the 30-mer template and 20-mer capture reagent at the onset of electrophoresis results in band blurring (lanes 2 and 4-6).

**FIG. 6B** is a representation of a denaturing PAGE gel following DNA-templated azide-to-amine transformation of azide 7 from **FIG. 5B****.** Lane 1 contains azide-linked 30-mer template + aldehyde-linked 20-mer capture reagent + 3 mM NaBH₃CN. Lane 2 contains azide-linked 30-mer template + phosphine-linked 10-mer reagent + aldehyde-linked 20-mer capture reagent + 3 mM NaBH₃CN. Lane 3 contains azide-linked 30-mer template + phosphine-linked 10-mer reagent containing a mismatched sequence + aldehyde-linked 20-mer capture reagent + 3 mM NaBH₃CN. Slight 50-mer captured product formation is observed in lanes 1 and 3, which arises from slow spontaneous reduction of the phenyl azide during the preparation of a substrate-linked template and during the DNA-templated reactions. The background reactivity observed in lanes 1 and 3 (<13 %) was subtracted to determine the reported yield for lane 2.

**FIG. 6C** is a representation of a denaturing PAGE gel following DNA-templated azide-to-carboxylic acid transformation of azide **8** from **FIG. 5B****.** Lane 1 contains azide-linked 30-mer template + amine-linked 20-mer capture reagent + 30 mM EDC + 15 mM sulfo-NHS. Lane 2 contains azide-linked 30-mer template + phosphine-linked 10-mer reagent + amine-linked 20-mer capture reagent + 30 mM EDC + 15 mM sulfo-NHS. Lane 3 contains azide-linked 30-mer template + phosphine-linked 10-mer reagent containing a mismatched sequence + amine-linked 20-mer capture reagent + 30 mM EDC + 15 mM sulfo-NHS.

**FIG. 6D** is a representation of a denaturing PAGE gel following DNA-templated azide-to-thiol transformation of azide **11** from **FIG. 5B** using a 10% polyacrylamide gel. Lane 1 contains azide-linked 30-mer template + alkyl bromide-linked 20-mer capture reagent. Lane 2 contains azide-linked 30-mer template + phosphine-linked 10-mer reagent + alkyl bromide-linked 20-mer capture reagent. Lane 3 contains azide-linked 30-mer template + phosphine-linked 10-mer reagent containing a mismatched sequence + alkyl bromide-linked 20-mer capture reagent.

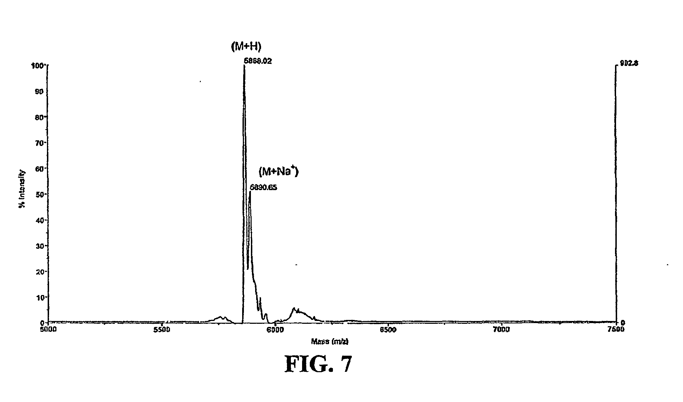

**FIG. 7** is a representative MALDI-TOF spectrum from a DNA-templated functional group transformation (in this case, the amine product arising from azide **1**).

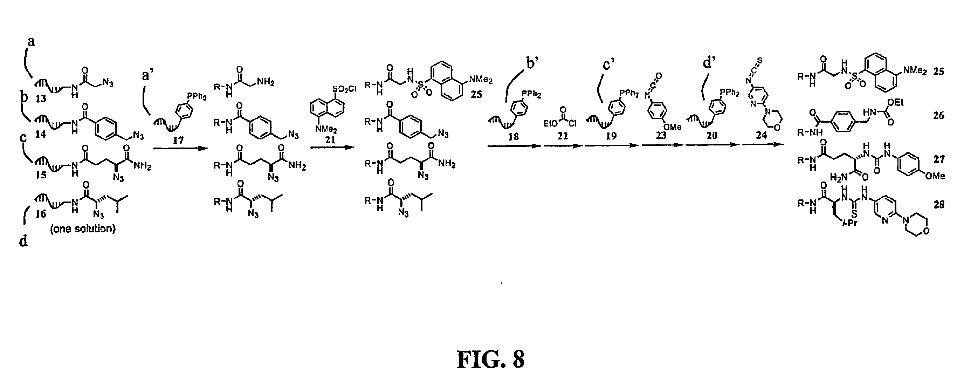

**FIG. 8** depicts a reaction of a single solution containing four azides with four non-DNA-linked small-molecule electrophiles to generate four sequence-programmed sulfonamide, carbamate, urea, and thiourea products. Template **13** is attached to an oligonucleotide having codon sequence **a.** The triphenylphosphine containing transfer unit **17** is attached to an oligonucleotide having anti-codon sequence **a'.** During templated synthesis codon sequence a anneals to anti-codon sequence **a'.** Similarly, templates **14, 15,** and **16** contain codon sequences **b, c,** and **d,** respectively, which anneal to transfer units **18, 19,** and **20** via anti-codon sequences **b', c',** and **d',** respectively.

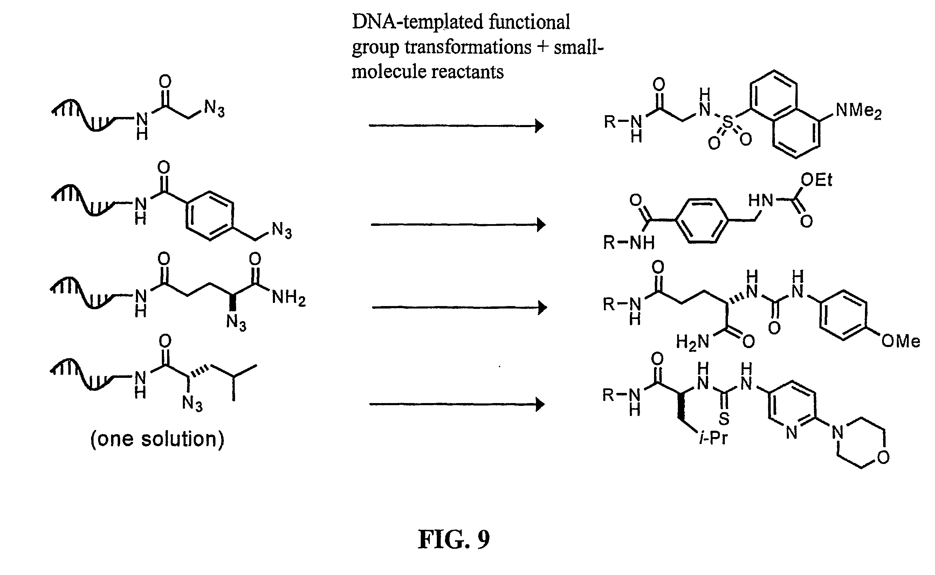

**FIG. 9** is a schematic illustration showing the starting reagents used and reaction products created in **FIG. 8****.**

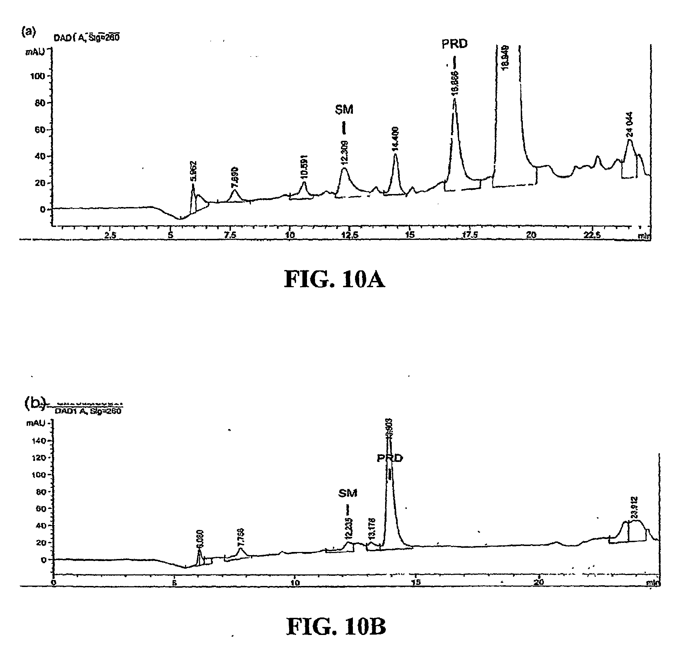

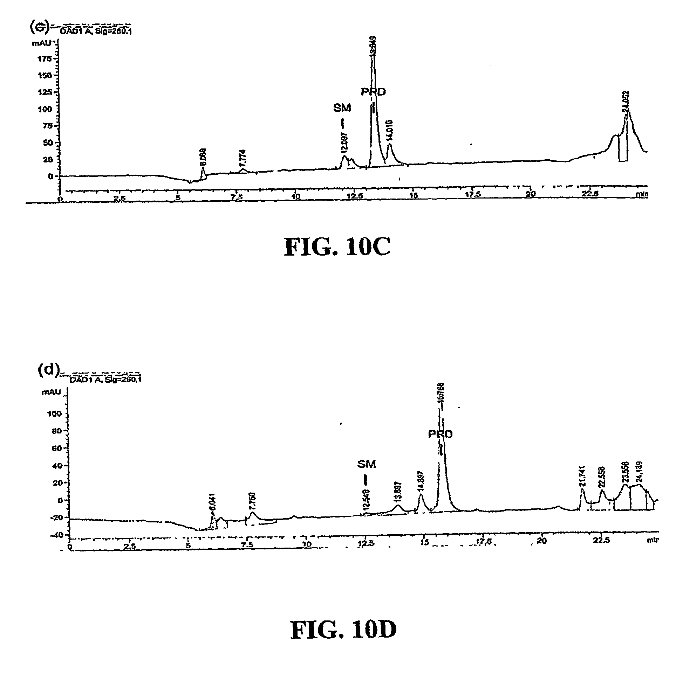

**FIG. 10A** through **FIG. 10D** are drawings of HPLC traces (monitored at 260 nm) following HPLC analysis of reactions of amine-linked templates with small-molecule reagents. SM indicates unreacted starting material amine-linked template peaks. PRD indicates derivatized products. Unless otherwise noted, peaks other than those labeled as SM or PRD do not correspond to DNA-linked species as judged by UV absorption at 230 nm and by MALDI-TOF analysis. **FIG. 10A** shows the results of the reaction of amine-linked template **13** with dansyl chloride **21** (reagents **13** and **21** are shown in **FIG. 8****).** **FIG.10B** shows the results of the reaction of amine-linked template **14** with ethyl chloroformate **22** (reagents **14** and **22** are shown in **FIG. 8****).** **FIG. 10C** shows the results of the reaction of amine-linked template **15** with 4-methoxy phenyl isocyanate **23** (reagents **15** and **23** are shown in **FIG. 8****).** **FIG. 10D** shows the results of the reaction of amine-linked template **16** with 6-morpholino pyridinyl 3-methoxyl phenyl isocyanate **24** (reagents **16** and **24** are shown in **FIG. 8****).**

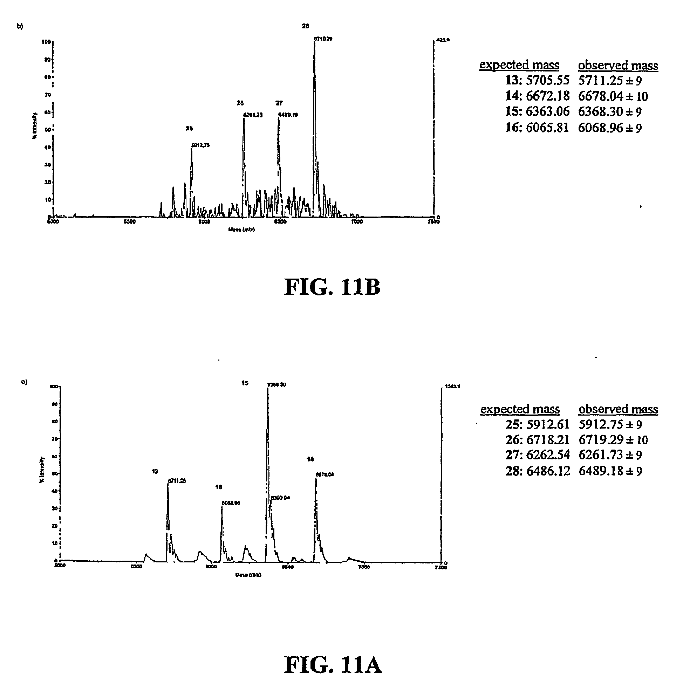

**FIG. 11A** shows a MALDI-TOF spectrum of the four azide starting materials in one solution (reagents **13-16** in **FIG. 8**). **FIG.11B** shows a MALDI-TOF spectrum of the four sequence-specific transformation products (products **25-28** in FIG. **8****)** of the azide starting materials **13-16.**

### DETAILED DESCRIPTION

The present invention is useful in the synthesis of libraries of molecules, for example, small molecules. The functional group transformations described herein are particularly useful in expanding the scope of nucleic acid-templated organic syntheses by addressing the need for reagents to be tethered to oligonucleotides. When the linkage of reagents to an oligonucleotide is not possible or convenient, these transformations allow such reagents to nevertheless contribute to small molecule syntheses while preserving the correspondence between oligonucleotide sequence and resulting product structure.

In one aspect, the invention provides a method of synthesizing a reaction product as set out in claim 1.

This approach is shown schematically in **FIG.1****.** Briefly, a first reactive unit (FRU) is reacted with a second, different reactive unit (SRU) to produce a reaction intermediate (RI). The RI is covalently attached, to an identifying sequence (IS). The IS is be an oligonucleotide (for example, DNA, or derivatives thereof, RNA, or derivatives thereof). Then, the RI-IS complex is combined with a free reactant (FR) under conditions to permit the FR to chemically transform the RI into a reaction product (RP). The RP is still linked to the IS, which can be used to identify RP and the synthetic history of RP.

In one approach, the IS, for example, a nucleic acid sequence defining a specific codon sequence or anti-codon sequence, is linked to the FRU prior to the reaction that produces the RI. The IS remains linked to the RI after the reaction so as to provide an IS linked to the RI. Following creation of the RP, the IS remains linked to the RP so that it is possible to identify the RP and its synthetic history. It is contemplated that the SRU may also be linked to a sequence complementary to the IS. As a result, during step (a), the IS hybridizes to the sequence complementary to the IS so as to bring the FRU and SRU into reactive proximity.

In another approach, the IS, for example, a nucleic acid sequence defining a specific codon sequence or anti-codon sequence, is linked to the RI after it has been formed by the reaction between FRU and SRU. The RI can then be chemically transformed via the FR into RP. The IS remains linked to the RP. The IS can be linked enzymatically, for example, by a polymerase or ligase, to the RI after formation of the RI.

In one embodiment, the FR is at least five times more reactive with the RI than with at least one of, and optionally all of, the reactive units or other reactive intermediates in the starting mixture. Furthermore, depending on the reactants and reaction conditions, in other embodiments the FR is at least ten times, at least fifty times, at least one hundred times, at least two hundred fifty times, at least five hundred times, or at least one thousand times more reactive with the RI than with at least one of, and optionally all of, the reactive units or other reactive intermediates in the starting mixture. In addition, depending upon the reactants and reaction conditions, the RP is synthesized with a yield greater than or equal to 50%, greater than or equal to 75%, greater than or equal to 85%, or greater than or equal to 98%.

The reactivity of the FR to the RI relative to the starting materials FRU and SRU can be determined experimentally. The amount of product produced by combining FR and RI under standard reaction conditions can be determined. The amount of product produced by combining in equimolar amounts FR with either FRU or SRU under the same reaction conditions can be determined. The yields of products can be determined by standard techniques in the chemical arts. Based on the relative amounts of product produced in each reaction it is possible to determine whether the FR is more reactive, and, if so, how much more reactive, than the FRU or the SRU. Similar approaches can be used to determine whether the free reactant is more reactive with one reaction intermediate than with other, different reactive intermediates.

In another aspect, the invention provides a method of synthesizing a reaction product via nucleic acid-templated synthesis as set out in claim 6.

This approach is shown schematically in **FIG. 2**, where a first reactive unit (FRU) is attached, for example, covalently attached, to a first oligonucleotide comprising a codon sequence (CS). The combination of the first reactive unit and the oligonucleotide can be referred to as a template. The codon sequence may identify the FRU, for example, like an IS. Alternatively, the CS may further comprise a separate identifier sequence (IS) that identifies the FRU. In the latter scenario, the CS can identify the second reactive unit (SRU) that reacts with the FRU to create the reaction intermediate (RI) and the IS can identify the FRU.

In this approach, the SRU is attached, for example, covalently attached to a second oligonucleotide that contains an anti-codon sequence (ACS) complementary to the CS. The combination of the second reactive unit with the anti-codon sequence can be referred to as a transfer unit. When the template and the transfer unit are combined under the appropriate reaction conditions, the CS and the ACS anneal to one another to bring the FRU and SRU into reactive proximity. The FRU and SRU then react with one another, for example, by proximity catalysis, to produce RI that is still linked to CS. When combined with the free reactant (FR), the RI is chemically transformed by FR into a reaction product (RP) that is still linked to the CS. Assuming that the oligonucleotide attached to RP contains the CS, then it is possible to determine what SRU was involved in the synthesis of the RI and/or the RP. Similarly; if the oligonucleotide attached to RP contains the IS, then it is possible to determine what FRU was involved in the synthesis of the RI and/or RP. Accordingly, this information can be used to determine the identity and synthetic history of RI and/or RP.

Furthermore, it is contemplated that the FRU can be a small molecule scaffold that can be used during nucleic acid-templated synthesis to produce a small molecule. In particular, the small molecule scaffold can be used as a core on which to assemble the substituents of the small molecule.

In one embodiment, the FR is at least five times more reactive with the RI than with at least one of, and optionally all of, the reactive units or other reactive intermediates in the starting mixture. Furthermore, depending on the reactants and reaction conditions, in other embodiments the FR is at least ten times, at least fifty times, at least one hundred times, at least two hundred fifty times, at least five hundred times, or at least one thousand times more reactive with the RI than with at least one of, and optionally all of, the reactive units or other reactive intermediates in the starting mixture. In addition, depending upon the reactants and reaction conditions, the RP is synthesized with a yield greater than or equal to 50%, greater than or equal to 75%, greater than or equal to 85%, or greater than or equal to 98%.

In another aspect, the invention provides a method of synthesizing a reaction product as set out in claim 7.

This approach is shown schematically in **FIG. 3** and is similar to the approach shown schematically in **FIG. 1** except that the first reaction unit is present as a mixture of first reactive units. Briefly, a starting mixture containing four first reactive units denoted FRU₁, FRU₂, FRU₃, and FRU₄ are combined with a second reactive unit denoted as SRU₁. Under the appropriate conditions, FRU₁ and SRU₁ react with one another to produce a reaction intermediate denoted as RI₁. An identifier sequence (IS) can be attached to the RI₁ which identifies RI₁. Thereafter, a free reactant (FR) is combined to the mixture under conditions for the RI to be chemically transformed into a reaction product denoted RP₁. The RP₁ is still linked to the IS which can be used to identify RP and the synthetic history of RP. In addition, it is contemplated that RP₁ can be exposed to other rounds of functional group transformations, especially where the FRU is a small molecule scaffold, to produce further modified products.

In another aspect, the invention provides a method of synthesizing a reaction product via nucleic acid-templated synthesis as set out in claim 13.

This approach is shown schematically in **FIG. 4** and is similar to the approach shown schematically in **FIG. 2** except the first reactive unit is present as a mixture of first reactive units. Briefly, the initial reaction contains a plurality of templates, where each template contains a first reactive unit (denoted as FRU₁, FRU₂, FRU₃ and FRU₄) attached, preferably, covalently attached, to its own respective oligonucleotide containing its own codon unique sequence (CS). The oligonucleotide preferably also contains an identifier sequence (IS) that identifies what first reactive unit is attached to what codon sequence of the template. A transfer unit containing a second reactive unit (SRU) attached, preferably, covalently attached, to an oligonucleotide containing an anti-codon sequence complementary to the codon sequence is combined with the templates. The ACS of the transfer unit anneals to the CS of the template to bring the FRU₁ and SRU into reactive proximity, whereupon the FRU₁ and SRU react with one another to produce reaction intermediate (RI₁) that still remains attached to the oligonucleotide containing the CS. When combined with the free reactant (FR), the RI is chemically transferred by the FR to produce the reaction product (RP) that is still linked to the CS. Assuming that the oligonucleotide attached to the RP contains a CS, then it is possible to determine what SRU was involved in the synthesis of RI and/or the RP. Similarly, if the oligonucleotide attached to RP contains the IS, then it is possible to determine what FRU was involved in the synthesis of the RI and/or the RP. Accordingly, this information can be used to determine the identity and synthetic history of RI and/or RP. As discussed, it is contemplated that the first reactive units can be small molecule scaffolds useful in the design and synthesis of a small molecule library.

In addition, it is possible that multiple different functional group transformations can occur simultaneously in the same reaction vessel. Accordingly, the method can also include the additional steps of: providing a third different reactive unit linked to a third oligonucleotide comprising an anti-codon sequence complementary to the codon sequence of at least one different first reactive unit, wherein the anti-codon sequence is indicative of the third reactive unit; annealing the codon sequence of a different one of the first oligonucleotides with the anti-codon sequence of the third oligonucleotide to induce a reaction between the first and third reactive units to form a second reaction intermediate attached at least to a first, different oligonucleotide; and combining the second reaction intermediate with a free reactant selectively reactive with the second reaction intermediate, thereby synthesizing a second reaction product attached to the identifying sequence, wherein the free reactant is more reactive with the second reaction intermediate than with at least one of, and optionally all of, the reactive units or other reactive intermediates in the mixture.

In addition, it is contemplated that the reaction products can be exposed to other rounds of functional group transformations, for example, where FRU₁ is a small molecule scaffold, to produce further modified products.

As will be appreciated by those skilled in the art, the method of the invention can be used to expand the range of chemistries that can be used during nucleic acid-templated chemical syntheses. General considerations concerning the selection and use of templates, transfer units, reaction conditions, reaction chemistries, selection procedures are know in the art. A general discussion of these considerations follows.

### I. TEMPLATE CONSIDERATIONS

The nucleic acid template can direct a wide variety of chemical reactions without obvious structural requirements by sequence-specifically recruiting reactants linked to complementary oligonucleotides. During synthesis, the template hybridizes or anneals to one or more transfer units to direct the synthesis of a reaction intermediate that can subsequently be converted by a free reactant into a reaction product. The reaction product then is selected or screened based on certain criteria, such as the ability to bind to a preselected target molecule. Once the reaction product has been identified, the associated template can then be sequenced to decode the synthetic history of the reaction intermediate and/or the reaction product.

### (i) Template Format

The length of the template may vary greatly depending upon the type of the nucleic acid-templated synthesis contemplated. For example, in certain embodiments, the template may be from 10 to 10,000 nucleotides in length, from 20 to 1,000 nucleotides in length, from 20 to 400 nucleotides in length, from 40 to 1,000 nucleotides in length, or from 40 to 400 nucleotides in length. The length of the template will of course depend on, for example, the length of the codons, the complexity of the library, the complexity and/or size of a reaction product, the use of spacer sequences, *etc.*

The template may incorporate a hairpin loop on one end terminating in a reactive unit that can interact with one or more reactive units associated with transfer units. For example, a DNA template can comprise a hairpin loop terminating in a 5'-amino group, which may or may not be protected. The amino group may act as an initiation point for formation of an unnatural polymer or small molecule.

### (ii) Codon Usage

It is contemplated that the sequence of the template may be designed in a number of ways. For example, the length of the codon must be determined and the codon sequences must be set. If a codon length of two is used, then using the four naturally occurring bases only 16 possible combinations are available to be used in encoding the library. If the length of the codon is increased to three (the number Nature uses in encoding proteins), the number of possible combinations increases to 64. If the length of the codon is increased to four, the number of possible combinations increases to 256. Other factors to be considered in determining the length of the codon are mismatching, frame-shifting, complexity of library, *etc.* As the length of the codon is increased up to a certain point the number of mismatches is decreased; however, excessively long codons likely will hybridize despite mismatched base pairs.

Although the length of the codons may vary, the codons may range from 2 to 50 nucleotides, from 2 to 40 nucleotides, from 2 to 30 nucleotides, from 2 to 20 nucleotides, from 2 to 15 nucleotides, from 2 to 10 nucleotides, from 3 to 50 nucleotides, from 3 to 40 nucleotides, from 3 to 30 nucleotides, from 3 to 20 nucleotides, from 3 to 15 nucleotides, from 3 to 10 nucleotides, from 4 to 50 nucleotides, from 4 to 40 nucleotides, from 4 to 30 nucleotides, from 4 to 20 nucleotides, from 4 to 15 nucleotides, from 4 to 10 nucleotides, from 5 to 50 nucleotides, from 5 to 40 nucleotides, from 5 to 30 nucleotides, from 5 to 20 nucleotides, from 5 to 15 nucleotides, from 5 to 10 nucleotides, from 6 to 50 nucleotides, from 6 to 40 nucleotides, from 6 to 30 nucleotides, from 6 to 20 nucleotides, from 6 to 15 nucleotides, from 6 to 10 nucleotides, from 7 to 50 nucleotides, from 7 to 40 nucleotides, from 7 to 30 nucleotides, from 7 to 20 nucleotides, from 7 to 15 nucleotides, from 7 to 10 nucleotides, from 8 to 50 nucleotides, from 8 to 40 nucleotides, from 8 to 30 nucleotides, from 8 to 20 nucleotides, from 8 to 15 nucleotides, from 8 to 10 nucleotides, from 9 to 50 nucleotides, from 9 to 40 nucleotides, from 9 to 30 nucleotides, from 9 to 20 nucleotides, from 9 to 15 nucleotides, from 9 to 10 nucleotides. Codons, however, preferably are 3, 4, 5, 6, 7, 8, 9 or 10 nucleotides in length.

A set of codons used in the template preferably maximizes the number of mismatches between any two codons within a codon set to ensure that only the proper anti-codons of the transfer units anneal to the codon sites of the template. Furthermore, it is important that the template has mismatches between all the members of one codon set and all the codons of a different codon set to ensure that the anti-codons do not inadvertently bind to the wrong codon set. The choice of exemplary codon sets and methods of creating functional codon sets are described, for example, in U.S. Patent Publication No. US 2004/0180412. Using this and other approaches, different sets of codons can be generated so that no codons are repeated.

When the nucleic acid-templated synthesis is used to produce a polymer or a small molecule, spacer sequences may also be placed between the codons to prevent frame shifting. For example, the bases of the template that encode a polymer subunit (the "genetic code" for the polymer) may be chosen so as to minimize the possibility of out-of-frame annealing. These genetic codes reduce undesired frameshifted nucleic acid-templated polymer translation and differ in the range of expected melting temperatures and in the minimum number of mismatches that result during out-of-frame annealing.

### (iii) Template Synthesis

The templates may be synthesized using methodologies well known in the art. These methods include both *in vivo* and *in vitro* methods including PCR, plasmid preparation, endonuclease digestion, solid phase synthesis (for example, using an automated synthesizer), *in vitro* transcription, strand separation, *etc.* Following synthesis, the template, when desired may be attached (for example, covalently or non covalently attached) with a reactive unit of interest using standard coupling chemistries known in the art.

An efficient method to synthesize a large variety of templates is to use a "split-pool" technique. The oligonucleotides are synthesized using standard 3' to 5' chemistries. First, the constant 3' end is synthesized. This is then split into *n* different vessels, where *n* is the number of different codons to appear at that position in the template. For each vessel, one of the *n* different codons is synthesized on the (growing) 5' end of the constant 3' end. Thus, each vessel contains, from 5' to 3', a different codon attached to a constant 3' end. The *n* vessels then are pooled, so that a single vessel contains *n* different codons attached to the constant 3' end. Any constant bases adjacent the 5' end of the codon are now synthesized. The pool then is split into *m* different vessels, where *m* is the number of different codons to appear at the next (more 5') position of the template. A different codon is synthesized (at the 5' end of the growing oligonucleotide) in each of the *m* vessels. The resulting oligonucleotides are pooled in a single vessel. Splitting, synthesizing, and pooling are repeated as required to synthesize all codons and constant regions in the oligonucleotides.

### II. TRANSFER UNITS

A transfer unit comprises an oligonucleotide containing an anti-codon sequence and a reactive unit. The anti-codons are designed to be complementary to the codons present in the template. Accordingly, the sequences used in the template and the codon lengths should be considered when designing the anti-codons. Any molecule complementary to a codon used in the template may be used, including natural or non-natural nucleotides. In certain embodiments, the codons include one or more bases found in nature (*i.e.*, thymidine, uracil, guanidine, cytosine, and adenine). Thus, the anti-codon can include one or more nucleotides normally found in Nature with a base, a sugar, and an optional phosphate group.

As discussed above, the anti-codon is associated with a particular type of reactive unit to form a transfer unit. The reactive unit may represent a distinct entity or may be part of the functionality of the anti-codon unit. In certain other embodiments, where a small molecule library is to be created rather than a polymer library, the anti-codon generally is associated with a reactive unit or reactant used to modify a small molecule scaffold. In certain embodiments, the reactant is linked to the anti-codon via a linker long enough to allow the reactant to come into reactive proximity with the small molecule scaffold. The linker preferably has a length and composition to permit intramolecular reactions but yet minimize intermolecular reactions. The reactants include a variety of reagents as demonstrated by the wide range of reactions that can be utilized in nucleic acid-templated synthesis and can be any chemical group, catalyst (*e.g.*, organometallic compounds), or reactive moiety (*e.g.*, electrophiles, nucleophiles) known in the chemical arts.

In certain embodiments, each anti-codon sequence is associated with one monomer type. For example, the anti-codon sequence ATTAG may be associated with a carbamate residue with an isobutyl side chain, and the anti-codon sequence CATAG may be associated with a carbamate residue with a phenyl side chain. This one-for-one mapping of anti-codon to monomer units allows the decoding of any polymer of the library by sequencing the nucleic acid template used in the synthesis and allows synthesis of the same polymer or a related polymer by knowing the sequence of the original polymer. By changing (*e.g.*, mutating) the sequence of the template, different monomer units may be introduced, thereby allowing the synthesis of related polymers, which can subsequently be selected and evolved. In certain preferred embodiments, several anti-codons may code for one monomer unit as is the case in Nature.

The anti-codon can be associated with the reactant through a linker moiety. The linkage can be cleavable by light, oxidation, hydrolysis, exposure to acid, exposure to base, reduction, *etc.* Fruchtel et al. (1996) ANGEW. CHEM. INT. ED. ENGL. 35: 17 describes a variety of linkages useful in the practice of the invention. The linker facilitates contact of the reactant with the small molecule scaffold and in certain embodiments, depending on the desired reaction, positions DNA as a leaving group ("autocleavable" strategy), or may link reactive groups to the template via the "scarless" linker strategy (which yields product without leaving behind an additional atom or atoms having chemical functionality), or a "useful scar" strategy (in which a portion of the linker is left behind to be functionalized in subsequent steps following linker cleavage). Useful linkers, their design and use are described in U.S. Patent Application Publication No. US 2004/0180412.

The specific annealing of transfer units to templates permits the use of transfer units at concentrations lower than concentrations used in many traditional organic syntheses. Thus, transfer units can be used at submillimolar concentrations (*e.g.* less than 100 µM, less than 10 µM, less than 1 µM, less than 100 nM, or less than 10 nM).

### III. CHEMICAL REACTIONS

A variety of compounds and/or libraries can be prepared using the methods described herein. In certain embodiments, compounds that are not, or do not resemble, nucleic acids or analogs thereof, are synthesized according to the method of the invention. In certain other embodiments, compounds that are not, or do not resemble, proteins, peptides, or analogs thereof, are synthesized according to the method of the invention.

### (i) Coupling Reactions for Small Molecule Synthesis

In some embodiments, it is possible to create compounds such as small molecules using the methods described herein. These small molecules may be like natural products, non-polymeric, and/or non-oligomeric. The substantial interest in small molecules is due in part to their use as the active ingredient in many pharmaceutical preparations although they may also be used, for example, as catalysts, materials, or additives.

In synthesizing small molecules using the method of the present invention, an evolvable template can be used. The template can include a small molecule scaffold upon which the small molecule is to be built, or a small molecule scaffold may be added to the template. The small molecule scaffold can be any chemical compound with two or more sites for functionalization. For example, the small molecule scaffold can include a ring system (*e.g.*, the ABCD steroid ring system found in cholesterol) with functionalizable groups coupled to the atoms making up the rings. In another example, the small molecule may be the underlying structure of a pharmaceutical agent such as morphine, epothilone or a cephalosporin antibiotic. The sites or groups to be functionalized on the small molecule scaffold may be protected using methods and protecting groups known in the art. The protecting groups used in a small molecule scaffold may be orthogonal to one another so that protecting groups can be removed one at a time.

In this approach, the transfer units comprise an anti-codon associated with a reactant or a building block for use in modifying, adding to, or taking away from the small molecule scaffold. The reactants or building blocks may be, for example, electrophiles (*e.g.*, acetyl, amides, acid chlorides, esters, nitriles, imines), nucleophiles (*e.g.*, amines, hydroxyl groups, thiols), catalysts (*e.g.*, organometallic catalysts), or side chains. The transfer units are allowed to contact the template under hydridizing conditions. As a result of oligonucleotide annealing, the attached reactant or building block is allowed to react with a site on the small molecule scaffold to produce one or more reaction intermediates. The reaction intermediates can then be reacted with a free reactant to produce a reaction product.

The reaction conditions, linker, reactant, and site to be functionalized are chosen to avoid intermolecular reactions and accelerate intramolecular reactions. Sequential or simultaneous contacting of the template with transfer units can be employed depending on the particular compound to be synthesized.

After the sites on the scaffold have been modified, the newly synthesized small molecule remains associated with the template that encoded its synthesis. Decoding the sequence of the template permits the deconvolution of the synthetic history and thereby the structure of the small molecule. The template can also be amplified in order to create more of the desired small molecule and/or the template can be evolved (mutagenized) to create related small molecules. The small molecule can also be cleaved from the template for purification or screening.

### (ii) Coupling Reactions for Polymer Synthesis

In certain embodiments, polymers, specifically unnatural polymers, can be prepared using the techniques described herein. Exemplary unnatural polymers include, but are not limited to, peptide nucleic acid (PNA) polymers, polycarbamates, polyureas, polyesters, polyacrylate, polyalkylene (*e.g.*, polyethylene, polypropylene), polycarbonates, polypeptides with unnatural stereochemistry, polypeptides with unnatural amino acids, and combination thereof. In certain embodiments, the polymers comprise at least 10, 25, 75, 100, 125, 150 monomer units or more. The polymers synthesized using the methodologies described herein may be used, for example, as catalysts, pharmaceuticals, metal chelators, or catalysts.

In preparing certain unnatural polymers, the monomer units attached to the anti-codons may be any monomers or oligomers capable of being joined together to form a polymer. The monomer units may be, for example, carbamates, D-amino acids, unnatural amino acids, PNAs, ureas, hydroxy acids, esters, carbonates, acrylates, or ethers.

### (iii) Reaction Conditions

It is understood that nucleic acid-templated reactions, for example, nucleic acid-templated reactions to produce reaction intermediates, can occur in aqueous or non-aqueous (*i.e.,* organic) solutions, or a mixture of one or more aqueous and non-aqueous solutions. In aqueous solutions, reactions can be performed at pH ranges from about 2 to about 12, or preferably from about 2 to about 10, or more preferably from about 4 to about 10. The reactions used in DNA-templated chemistry preferably should not require very basic conditions (*e.g.*, pH > 12, pH > 10) or very acidic conditions (*e.g.*, pH < 1, pH < 2, pH < 4), because extreme conditions may lead to degradation or modification of the nucleic acid template and/or molecule (for example, the polymer, or small molecule) being synthesized. The aqueous solution can contain one or more inorganic salts, including, but not limited to, NaCl, Na₂SO₄, KCl, Mg⁺², Mn⁺², *etc.,* at various concentrations.

Organic solvents suitable for nucleic acid-templated reactions include, but are not limited to, methylene chloride, chloroform, dimethylformamide, and organic alcohols, including methanol and ethanol. To permit quantitative dissolution of reaction components in organic solvents, quatemized ammonium salts, such as, for example, long chain tetraalkylammonium salts, can be added (Jost et al. (1989) NUCLEIC ACIDS RES. 17: 2143; Mel'nikov et al. (1999) LANGMUIR 15: 1923-1928).

Nucleic acid-templated reactions may require a catalyst, such as, for example, homogeneous, heterogeneous, phase transfer, and asymmetric catalysis. In other embodiments, a catalyst is not required. The presence of additional, accessory reagents not linked to a nucleic acid are preferred in some embodiments. Useful accessory reagents can include, for example, oxidizing agents (*e.g.*, NaIO₄); reducing agents (*e.g.*, NaCNBH₃); activating reagents (*e.g.*, EDC, NHS, and sulfo-NHS); transition metals such as nickel (*e.g.,* Ni(NO₃)₂), rhodium (*e.g.* RhCl₃), ruthenium (*e.g.* RuCl₃), copper (*e.g.* Cu(NO₃)₂), cobalt (*e.g.* CoCl₂), iron (*e.g.* Fe(NO₃)₃), osmium (*e.g.* OsO₄), titanium (*e.g.* TiCl₄ or titanium tetraisopropoxide), palladium (*e.g.* NaPdCl₄), or Ln; transition metal ligands (*e.g.*, phosphines, amines, and halides); Lewis acids; and Lewis bases.

Reaction conditions preferably are optimized to suit the nature of the reactive units and oligonucleotides used. It is understood that the choice of reagents, for example, free reactants, and the reaction conditions used to create the reaction intermediates and to convert the reaction intermediates into final products will depend upon the particular compounds and libraries to be produced. It is contemplated, however, that the choice of reagents and reaction conditions is within the level of skill in the art.

### (iv) Classes of Chemical Reactions

It is understood that a large variety of chemical reactions can be used to create the reaction intermediates and/or to create the reaction products from the reaction intermediates. Known chemical reactions for synthesizing polymers, small molecules, or other molecules can be used in nucleic acid-templated reactions. Thus, reactions such as those listed in *March's Advanced Organic Chemistry, Organic Reactions, Organic Syntheses,* organic text books, journals such as *Journal of the American Chemical Society, Journal of Organic Chemistry, Tetrahedron, etc.,* and Carruther's *Some Modern Methods of Organic Chemistry* can be used. The chosen reactions preferably are compatible with nucleic acids such as DNA or RNA or are compatible with the modified nucleic acids used as the template.

Notwithstanding the foregoing, it is contemplated that the invention is particularly useful in performing certain functional group transformations, which include, without limitation, azide-to-amine transformations, azide-to-thiol transformations, azide-to-carboxylic acid transformations, hydroxyl-to-amine transformations, hydroxyl-to-thiol transformations, acetal-to-aldehyde transformations, ketal-to-ketone transformations, carbonate-to-hydroxyl group transformations, carbamate-to-amine transformations, thiocarbonate-to-thiol transformations, nitro group-to-amine transformations, sulfonamide-to-amine transformations, alkene-to-epoxide transformations, α,β-unsaturated ketone-to-epoxide transformations, epoxide-to-1,2-diol transformations, epoxide-to-1,2-hydroxy amine transformations, epoxide-to-1,2-hydroxy sulfide transformations, alkene-to-aziridine transformations, aziridine-to-1,2-diamine transformations, aziridine-to-1,2-amino sulfide transformations, phosphonate ester-to-phosphonic acid transformations, imide-to-amine transformations, and nitrile-to-carboxylic acid transformations. Other exemplary transformations may be found, for example, in Greene et al. (ed.) (1999) PROTECTIVE GROUPS IN ORGANIC SYNTHESIS 3RD ED., Wiley-Interscience, and in Kocienski (1994) PROTECTING GROUPS, Thieme.

### IV. SELECTION, SCREENING AND IDENTIFICATION OF PRODUCTS

### (i) Selection and Screening Approaches

Selection and/or screening for reaction products with desired activities (such as catalytic activity, binding affinity, or a particular effect in an activity assay) may be performed using methodologies known and used in the art. For example, affinity selections may be performed according to the principles used in library-based selection methods such as phage display, polysome display, and mRNA-fusion protein displayed peptides. Selection for catalytic activity may be performed by affinity selections on transition-state analog affinity columns (Baca et al. (1997) PROC. NATL. ACAD. SCI. USA 94(19): 10063-8) or by function-based selection schemes (Pedersen et al. (1998) PROC. NATL. ACAD. SCI. USA 95(18): 10523-8). Since minute quantities of DNA (∼10⁻²⁰ mol) can be amplified by PCR (Kramer et al. (1999) CURRENT PROTOCOLS IN MOLECULAR BIOLOGY (ed. Ausubel, F. M.) 15.1-15.3, Wiley), these selections can be conducted on a scale ten or more orders of magnitude less than that required for reaction analysis by current methods, making a truly broad search both economical and efficient.

The templates and reaction products can be selected (or screened) for binding to a target molecule. In this context, selection or partitioning means any process whereby a library member bound to a target molecule is separated from library members not bound to target molecules. Selection can be accomplished by various methods known in the art.

The templates of the present invention contain a built-in function for direct selection and amplification. In most applications, binding to a target molecule preferably is selective, such that the template and the resulting reaction product bind preferentially with a specific target molecule, perhaps preventing or inducing a specific biological effect. Ultimately, a binding molecule identified using the present invention may be useful as a therapeutic and/or diagnostic agent. Once the selection is complete, the selected templates optionally can be amplified and sequenced. The selected reaction products, if present in sufficient quantity, can be separated from the templates, purified (*e.g.*, by HPLC, column chromatography, or other chromatographic method), and further characterized.

The selection strategy can be carried out to allow selection against almost any target. Importantly, the selection strategy does not require any detailed structural information about the target molecule or about the molecules in the libraries. The entire process is driven by the binding affinity involved in the specific recognition and binding of the molecules in the library to a given target.

The linkage between the template molecule and reaction product allows rapid identification of binding molecules using various selection strategies. Nucleic acid-templated syntheses broadly permit identifying binding molecules for any known target molecule. In addition, novel unknown targets can be discovered by isolating binding molecules against unknown antigens (epitopes) and using these binding molecules for identification and validation.

Selection of binding molecules from a library can be performed in any format to identify optimal binding molecules. Binding selections typically involve immobilizing the desired target molecule, adding a library of potential binders, and removing non-binders by washing. When the molecules showing low affinity for an immobilized target are washed away, the molecules with a stronger affinity generally remain attached to the target. The enriched population remaining bound to the target after stringent washing is preferably eluted with, for example, acid, chaotropic salts, heat, competitive elution with a known ligand or by proteolytic release of the target and/or of template molecules. The eluted templates are suitable for PCR, leading to many orders of amplification, whereby essentially each selected template becomes available at a greatly increased copy number for cloning, sequencing, and/or further enrichment or diversification.

The target molecule (peptide, protein, DNA or other antigen) can be immobilized on a solid support, for example, a container wall, a wall of a microtiter plate well. The library preferably is dissolved in aqueous binding buffer in one pot and equilibrated in the presence of immobilized target molecule. Non-binders are washed away with buffer. Those molecules that may be binding to the target molecule through their attached DNA templates rather than through their synthetic moieties can be eliminated by washing the bound library with unfunctionalized templates lacking PCR primer binding sites. Remaining bound library members then can be eluted, for example, by denaturation.

Alternatively, the target molecule can be immobilized on beads, particularly if there is doubt that the target molecule will adsorb sufficiently to a container wall, as may be the case for an unfolded target eluted from an SDS-PAGE gel. The derivatized beads can then be used to separate high-affinity library members from nonbinders by simply sedimenting the beads in a benchtop centrifuge. Alternatively, the beads can be used to make an affinity column. In such cases, the library is passed through the column one or more times to permit binding. The column then is washed to remove nonbinding library members. Magnetic beads are essentially a variant on the above; the target is attached to magnetic beads which are then used in the selection.

Library members that bind a target molecule can be released by denaturation, acid, or chaotropic salts. Alternatively, elution conditions can be more specific to reduce background or to select for a desired specificity. Elution can be accomplished using proteolysis to cleave a linker between the target molecule and the immobilizing surface or between the reaction product and the template. Also, elution can be accomplished by competition with a known competitive ligand for the target molecule. Alternatively, a PCR reaction can be performed directly in the presence of the washed target molecules at the end of the selection procedure. Thus, the binding molecules need not be elutable from the target to be selectable since only the template is needed for further amplification or cloning, not the reaction product itself. Indeed, some target molecules bind the most avid ligands so tightly that elution would be difficult.

### (ii) Identification of Products

Once all rounds of selection are complete, the templates which are, or formerly were, attached to the selected reaction product preferably are amplified using any suitable technique to facilitate sequencing or other subsequent manipulation of the templates. Natural oligonucleotides can be amplified by any state of the art method. These methods include, for example, polymerase chain reaction (PCR); nucleic acid sequence-based amplification (see, for example, Compton (1991) NATURE 350: 91-92), amplified anti-sense RNA (see, for example, van Gelder et al. (1988) PROC. NATL. ACAD. SCI. USA 85: 77652-77656); self-sustained sequence replication systems (Gnatelli et al. (1990) PROC. NATL. ACAD. SCI. USA 87: 1874-1878); polymerase-independent amplification (see, for example, Schmidt et al. (1997) NUCLEIC ACIDS RES. 25: 4797-4802, and *in vivo* amplification of plasmids carrying cloned DNA fragments. Descriptions of PCR methods are found, for example, in Saiki et al. (1985) SCIENCE 230: 1350-1354; Scharf et al. (1986) SCIENCE 233: 1076-1078; and in U.S. Patent No. 4,683,202. Ligase-mediated amplification methods such as Ligase Chain Reaction (LCR) may also be used. In general, any means allowing faithful, efficient amplification of selected nucleic acid sequences can be employed in the method of the present invention. It is preferable, although not necessary, that the proportionate representations of the sequences after amplification reflect the relative proportions of sequences in the mixture before amplification.

For non-natural nucleotides the choices of efficient amplification procedures are fewer. As non-natural nucleotides can be incorporated by certain enzymes including polymerases it will be possible to perform manual polymerase chain reaction by adding the polymerase during each extension cycle.

Once amplified, the sequences of the template that encoded a product of interest can be determined. Sequencing, for example, can be performed by a standard dideoxy chain termination method, or by chemical sequencing, for example, using the Maxam-Gilbert sequencing procedure. Alternatively, the sequence of the template (or, if a long template is used, the variable portion(s) thereof) can be determined by hybridization experiments. For example, a single-stranded template molecule associated with a detectable moiety such as a fluorescent moiety is exposed to a chip bearing a large number of clonal populations of single-stranded nucleic acids or nucleic acid analogs of known sequence, each clonal population being present at a particular addressable location on the chip. The template sequences are permitted to anneal to the chip sequences. The position of the detectable moieties on the chip then is determined. Based upon the location of the detectable moiety and the immobilized sequence at that location, the sequence of the template can be determined. It is contemplated that large numbers of such oligonucleotides can be immobilized in an array on a chip or other solid support.

Libraries can be evolved by introducing mutations at the DNA level, for example, using error-prone PCR (Cadwell et al. (1992) PCR METHODS APPL. 2: 28) or by subjecting the DNA to *in vitro* homologous recombination (Stemmer (1994) PROC. NATL. ACAD. SCI. USA 91: 10747; Stemmer (1994) NATURE 370: 389) or by cassette mutagenesis. Template evolution and evolutionary synthesis are described, for example, in U.S. Patent Application, Publication No. US 2004/0180412.

The following examples contain important additional information, exemplification and guidance that can be adapted to the practice of this invention in its various embodiments. Practice of the invention will be more fully understood from these following examples, which are presented herein for illustrative purpose only, and should not be construed as limiting in anyway.

### EXAMPLES

The following examples demonstrate the feasibility of sequence programmed functional group transformations. Examples 1 and 2 describe three sequence-programmed functional group transformations, namely-azide to-amine, azide-to-thiol, and azide-to-carboxylic acid transformations where the end products of the transformations have been characterized by gel electrophoresis (Example 1) or by mass spectrometry (Example 2). Example 3 shows that it is possible to transform amine-linked templates into a sulfonamide, a carbamate, a urea or a thiourea using small molecule reagents, for example, sulfonyl chloride, chloroformate, isocyanate, and isothiocyanate reactants not linked to DNA. Example 4 shows that it is possible to sequence-specifically transform, in a single-solution, a mixture of organic azides into amine intermediates and then sequence-specifically transform the amine intermediates into sulfonamide, carbamate, urea, and thiourea products using free reactants (e.g., sulfonyl chloride, chloroformate, isocyanate, and isothiocyanate) not linked to DNA.

### Example 1. DNA-Templated Transformation of Azides into Primary Amines, Carboxylic Acids, and Thiols (Characterization by PAGE)

This example describes sequence-programmed functional group transformations where an azide can be specifically converted into an amine, a thiol, or a carboxylic acid. The individual reaction schemes and the resulting reaction yields are shown in **FIGS. 5A** and **5B****.**

### I. Materials and Methods

### (i) Synthesis of Azido Acids

Azido substrates for the synthesis of compounds **1-12** shown in **FIG. 5B** were prepared from the corresponding carboxylic acid precursors as follows:

Azido Acetic Acid (Used to produce template **1** in **FIG. 5B****).** This reagent was produced as described in Lundquist et al. (2001) ORG LETT. 3: 781. The product was found to have the following characteristics: ¹H NMR (300 MHz, CDCl₃) 6 3.96 (2H, s).

Azido-3-Methyl Pentanoic Acid (Used to produce template **2** in **FIG. 5B****).** This reagent was produced as described in Lundquist *et al.* (2001) *supra.* The product was found to have the following characteristics: ¹H NMR (300 MHz, CDCl₃) δ 3.92(1H, br), 3.88 (2H, dd, *J* = 5.7 Hz, *J*= 9 Hz), 1.77 (2H, m), 0.99 (6H, t, *J*= 6.6 Hz).

4-Azidomethylbenzoic Acid (Used to prepare template **3** in **FIG. 5B****).** Sodium azide (1.3 g, 20 mmol) and 18-crown-6 ether (0.2 mL, 1 mmol) were dissolved in DMSO (4 mL). To the resulting solution was added 4-chloromethyl benzoic acid (1.71g, 10mmol) and the reaction mixture was stirred 12 h at 25 °C. The reaction was diluted in EtOAc, washed with 0.1 N HCl (2x), then washed with brine. The organic layer was dried with Na₂SO₄ and concentrated to provide a white solid (1.75 g, quant.). The resulting product was found to have the following characteristics: ¹H NMR (300 MHz, CDCl₃) δ 8.15 (2H, d, *J=* 8.4 Hz) 7.45 (2H, d, *J=* 8.4 Hz) (1H, s); ESMS calculated for C₈H₆N₃O₂: 176.0460; observed: 176.0461.

1-Azidocyclohexyl Carboxylic Acid (Used to prepare template **5** in **FIG. 5B****)** and Azidoisoglutamic Acid (Used to prepare template **4** in **FIG. 5B****)** were synthesized according to the method described for the synthesis of azido acids by diazo transfer in Lundquist *et al.* (2001) *supra.* 1-azidocyclohexyl carboxylic acid was found to have the following characteristics: ¹H NMR (300 MHz, CDCl₃) δ 1.86 (4H, m) 1.63 (4H, m) 1.36 (2H, m); CIMS calculated for C₇H₇N₃O₂ (M+NH₄⁺): 1187.1195; observed: 1187.1188. Azidoisoglutamic acid was found to have the following characteristics: ¹H NMR (300 MHz, CDCl₃) δ 6.43 (2H, d, ***J* =** 17.4 Hz) 3.14 (1H, dd, *J* = 14.1 Hz, *J*= 6.9 Hz) 2.54 (2H, t *J*= 7.5 Hz) 2.23 (2H, dd, *J*= 13.6 Hz *J* = 6.6 Hz); CIMS calculated for C₇H₇N₃O₂ (M+NH₄⁺): 190.0931; observed: 190.040.

1-Azido Methyl Benzoic Acid (Used to produce template **6** in **FIG. 5B****).** This reagent was produced as described in Wada et al. (2001) TETRAHEDRON LETT. 42: 1069-72, and also in Love et al. (2001). J. ORG CHEM. 66: 68165-76. The product was found to have the following characteristics: ¹H NMR (300 MHz, CDCl₃) δ 8.18 (1H, dd, *J* = 7.6 Hz, *J* = 1.2 Hz), 7.63 (1H, td, *J* = 7.5 Hz, *J* = 1.2 Hz), 7.55 (1H, d, *J* = 6.6 Hz), 7.46 (1H, td, *J* = 7.5 Hz, *J* = 1.5 Hz), 4.894 (2H, s).

4-Azidobenzoic Acid (Used to produce template **7** in **FIG. 5B****).** This reagent was purchased from Sigma-Aldrich (St. Louis, MO).

4-Azidobenzyl-Cyclohexyl Dicarboxylic Acid Monoester (Used to prepare template **8** in **FIG. 5B****).** *Trans-*cyclohexyl dicarboxylic acid (200 mg, 1.16 mmol), EDC (223 mg, 2.32 mmol), and N,N-diisopropylethylamine (0.4 mL, 2.32 mmol) were dissolved in CH₂Cl₂ (4 mL) and stirred for 30 min at 25°C. To this mixture was added 4-azido benzyl alcohol (86.6 mg, 0.58 mmol). The reaction was stirred 12 h at 25°C. The reaction mixture was concentrated and purified by flash chromatography (30% EtOAc/hexanes). The desired ester was obtained as a yellow solid (18.2 mg, 5%). The resulting product was found to have the following characteristics: ¹H NMR (300 MHz, CDCl₃) δ 7.34 (2H, d, *J* = 8.4 Hz) 7.03 (2H, d, *J*= 8.4 Hz) 5.08 (1H, s) 2.33 (2H, m) 2.09(4H, d, *J* = 9.3 Hz) 1.47 (4H, t, *J* = 9.9 Hz); ESMS calculated for C₁₅H₁₇N₃O₄ (M+HCO₂⁻): 348.1196; observed: 348.1195.

4-Azidobenzyl-Succinic Acid Monoester (Used to prepare template **9** in **FIG. 5B****).** 4-Azidobenzyl alcohol (100 mg, 0.67 mmol), succinic anhydride (134 mg, 1.37 mmol), and N,N-dimethylaminopyridine (3.7 mg, 30 µmol) were dissolved in DMF (1 mL) and stirred 12 h at 25 °C. The reaction mixture was concentrated and purified by flash chromatography (30% EtOAc/hexanes). The desired ester was obtained as yellow solid (75.9 mg, 45%). The resulting product was found to have the following characteristics: ¹H NMR (300 MHz, CDCl₃) δ 7.28 (2H, d, *J* = 7.8 Hz) 6.96 (2H, d, *J* = 7.8 Hz), 5.05 (2H, s), 2.63 (4H, m); ESMS calculated for C₇H₆N₃O₂: 248.0672; observed: 248.0660.

4-Azidobenzyl-Diphenicacid Monoester (Used to prepare template **10** in **FIG. 5B****).** 4-Azidobenzyl alcohol (112 mg, 0.5 mmol) and diphenic acid anhydride (74.5 mg, 0.5 mmol) were dissolved in pyridine (1 mL) and stirred 12 h at 25 °C. The reaction mixture was diluted in EtOAc, washed with phosphate buffer (pH 6.0, 2x), then washed with brine. The organic layer was dried over Na₂SO₄ and concentrated *in vacuo.* The crude product was purified by flash chromatography (25% EtOAc/hexanes). The desired ester was obtained as yellow solid (193 mg, 99%.). The resulting product was found to have the following characteristics: ¹H NMR (300 MHz, CDCl₃) δ 9.87 (1H, s) 7.92 (1H, dd, *J* = 7.2 Hz, *J* = 1.2 Hz) 7.86 (1H, dd, *J* = 7.8 Hz, *J* = 1.2 Hz) 7.43 (2H, dd, *J* = 5.7 Hz *J* = 1.5 Hz) 7.38 (2H, dd, *J* = 5.7 Hz, *J* = 1.2 Hz) 7.32 (2H, dd, *J* = 7.5 Hz, *J* = 1.2 Hz) 7.28 (2H, dd, *J* = 7.3 Hz, *J* = 1.2 Hz) 6.98 (2H, *J* = 8.4 Hz) 6.85 (2H, *J* = 8.4 Hz) 4.91(2H, *J* = 2.7 Hz); ESMS calculated for C₂₁H₁₆N₃O₄ (M+H⁺): 374.1141; observed: 374.1149.

1-Azidomethylbenzoyl Thio Acetic Acid Thioester (Used to prepare template **11** in **FIG. 5B****).** 2-Azidomethylbenzoyl acid (40 mg, 0.23 mmol) was mixed with EDC (64.9 mg, 0.34 mmol) and N-hydroxysuccinimide (NHS) (39.1 mg, 0.34 mmol) in CH₂Cl₂ at 25°C for 2 h. The reaction mixture was washed with NaHCO₃ (2x), then washed with brine. The organic layer was concentrated and the crude product was directly used in the next step without further purification. *N*-hydroxylsuccinimidyl 2-azidomethyl benzoate ester (16.4 mg, 47 µmol) and thioacetic acid (3.2 µL) in DMF (250 µL) were allowed to react at 25 °C for 24 h. The reaction mixture was diluted in EtOAc and washed with NaHCO₃ (2x), then washed with brine. The organic layer was dried with Na₂SO₄ and concentrated in vacuo. The crude mixture was purified by flash chromatography (30% EtOAc/hexanes) to provide the thioester (9.8 mg, 83%). The resulting product was found to have the following characteristics: ¹H NMR (300 MHz, CDCl₃) δ 7.95 (d, 1H, *J* = 7.8 Hz) 7.56 (t, 1H, *J* = 7.8 Hz) 7.50 (d, 1H, *J* = 6.6 Hz) 7.41 (t, 1H, *J* = 7.8 Hz) 4.64 (s, 2H) 3.88 (s, 2H); ESMS calculated for C₇H₆N₃O₂: 250.0287; observed: 250.0284.

2-Azidomethylbenzoyl Thio Propionic Acid Thioester (Used to prepare template **12** in **FIG. 5B****).** 2-azidomethylbenzoyl *N*-hydroxy succinimidyl (NHS) ester was prepared by mixing equal volumes of 1-azido methyl benzoic acid (used to produce template **6** in **FIG. 5B****)** (900 mM in DMF), EDC (900 mM in DMF) and NHS (900 mM in DMF) at 25 °C for 1 h. The thiol group was attached to the DNA oligonucleotide in a parallel preparation, and was incorporated into the template upon template formation (see below, preparation for 5' 2-azidomethylbenzoyl thio propionic acid thioester-linked DNA).

### (ii) Preparation of Functionalized Oligonucleotides

Throughout this Example and the following Examples, oligonucleotides were synthesized on a Perseptive Biosystems Expedite 8090 DNA synthesizer using standard phosphoramidite protocols and purified using preparative scale reverse-phase HPLC. Reagents for automated solid-phase oligonucleotide synthesis were purchased from Glen Research. For amine-terminated and biotinylated DNA oligonucleotides described below, 5' amino-modifier 5 (Glen Research) was used to prepare 5' amino-modified oligonucleotides; 3' amino-modifier C7 CPG (Glen Research) was used to prepare 3' amino-modified oligonucleotides; and biotin TEG CPG (Glen Research) was used to prepare 3' biotin-labeled oligonucleotides. Functionalized DNA oligonucleotides were purified by analytical scale reverse-phase HPLC.

Concentrations of purified oligonucleotides in solution were determined based on their absorbance at 260 nm measured on a Hewlett-Packard 8453 UV-visible spectrophotometer (Agilent Technologies). Oligonucleotides stained with ethidium bromide were visualized and quantitated by UV transillumination and densitometry using an Eagle Eye II densitometer (Stratagene).

### (a) Template Oligonucleotides

5' Azide-Linked DNA Oligonucleotide Templates (Used to produce templates **1-11** in **FIG. 5B****).** The *N-*hydroxy succinimidyl (NHS) ester of the desired azido acid was prepared by mixing equal volumes of the respective azido acid (900 mM in DMF), EDC (900 mM in DMF) and NHS (900 mM in DMF) at 25°C for 1 h. The crude NHS ester was added in two portions (50 µL each) to a solution containing 5' amino-modified DNA oligonucleotide (50 µL, typically 300 µM) in 100 mM sodium phosphate buffer (pH 7.2, 350 µL). The 30-mer template used in these preparations was ·5'NH₂(C₂H₄O)₂-PO₃H-GGT ACG AAT TGC ACT CGG GAA ATC CAC CTT (SEQ ID NO: 1). The coupling reaction was performed at 25 °C for 1 h. The resulting reaction mixture was directly loaded onto a NAP-5 size exclusion column (Amersham Biosciences) to remove organic solvent, salts, and excess small molecules, and the azide-linked DNA oligonucleotides were further purified by analytical scale reverse-phase HPLC (8-30% MeCN/0.1 M TEAA gradient). The desired oligonucleotide products were characterized by MALDI-TOF mass spectrometry.

5' 2-Azidomethylbenzoyl Thio Propionic Acid Thioester-Linked DNA (Used to produce template **12** in **FIG. 5B****).** A solution of 2,2'-dithiodipropionic acid in DMF (900 mM) was mixed with equal volumes of EDC (900 mM in DMF) and NHS (900 mM in DMF) at 25°C for 1 h. The crude NHS ester (50 µL) was added to a solution containing 5' amino-modified DNA oligonucleotide (50 µL, typically 300 µM) in 100 mM sodium phosphate buffer (pH 7.2, 350 µL). The coupling reaction was performed at 25 °C for 1 h. The reaction mixture was directly loaded onto a NAP-5 size exclusion column (Amersham Biosciences) and purified by analytical scale reverse-phase HPLC (8-30% MeCN/0.1 M TEAA gradient). The disulfide-linked oligonucleotide product was characterized by MALDI-TOF mass spectrometry. The 2-thiopropionic acid-linked oligonucleotide was prepared by treating the disulfide-linked oligonuleotide above (typically 10 µM) in 100 mM CAPS buffer (pH 8) with 20 mM DTT at 25°C for 0.5 h. Excess DTT was removed by passing the reaction mixture through a gel filtration column. In parallel, 2-azidomethylbenzoyl *N*-hydroxy succinimidyl (NHS) ester was prepared as described (see above, preparation for 2-Azidomethylbenzoyl Thio Propionic Acid Thioester). The crude NHS ester (100 µL) was added to a solution of 5' thiol-linked oligonucleotide (100 µL) in 100 mM sodium phosphate buffer (pH 7.2, 300 µL). The coupling reaction was performed at 25°C for 1 h. The reaction mixture was directly loaded onto a NAP-5 size exclusion column and purified by analytical scale reverse-phase HPLC (8-30% MeCN/0.1 M TEAA gradient). The desired oligonucleotide product was characterized by MALDI-TOF mass spectrometry.

### (b) Transfer Units

3' Triphenylphosphine-Linked DNA. Attachment of the triphenylphosphine group was performed on 3' amino-modified oligonucleotides linked to CPG resin. A 10-mer reagent fully complementary to the template had the structure 5' AAT TCG TAC C-OPO₃H-CH₂CH(CH₂OH)(CH₂)₄NHCOC₆H₄PPh₂ (SEQ ID NO: 2). A 10-mer reagent containing a three-base mismatch relative to the template had the following structure - 5' AAT ACA TCC C-OPO₃H-CH₂CH(CH₂OH)(CH₂)₄NHCOC₆H₄PPh₂ (SEQ ID NO: 3). The latter was used as a control in these experiments.

The Fmoc group on 3' FMOC-NH-oligonucleotides was removed by three cycles of: (*i*) treatment with 20% piperidine in DMF for 10 min; (*ii*) washing with DMF; and (*iii*) washing with MeCN. The resin was dried under a stream of nitrogen gas. A solution of 4-diphenylphosphino benzoic acid (30.6 mg, 100 µmol), EDC (19.1 mg, 100 µmol), *N,N-*diisopropylethylamine (36.8 µL, 211 µmol) in DMF (0.6 mL) was added to the resin and the mixture was incubated at 37 °C for 2 h. The resin was washed with DMF (2x) and with MeCN (2x), then dried under nitrogen. The derivatized oligonucleotide was cleaved from the CPG resin by incubation in 1:1 ammonium hydroxide:methyl amine (AMA) with tris(2-carboxyethyl)phosphine hydrochloride (TCEP-HCl, 1 mg) at 55 °C for 45 min. The cleavage solution was filtered and purified by analytical scale reverse-phase HPLC (8-30% MeCN/0.1 M TEAA gradient). The desired oligonucleotide products were characterized by MALDI-TOF mass spectrometry.

### (c) Capture Reagents

In order to perform polyacrylamide gel electrophoresis, the reaction products were captured using 20-mer secondary reagents (capture reagents) that annealed to the template.

Amino-modified DNA (This reagent was used to capture the products of the templates **8-10).** The 20-mer secondary reagent contained the sequence 5' TCC CGA GTG CAA TTC GTA CC-OPO₃H-CH₂CH(CH₂OH)(CH₂)₄NH₂ (SEQ ID NO: 4). This oligonucleotide was used as a starting material for the following capture reagents.

3' Bromoacetate-Linked DNA (This reagent was used to capture the products of the templates **11** and **12).** The NHS ester of bromoacetic acid was prepared by mixing equal volumes of 900 mM bromoacetic acid in DMF, 900 mM EDC in DMF, and 900 mM NHS in DMF at 25 °C for 1 h. The crude NHS ester (100 µL) was added to a solution of 5' amino-modified DNA oligonucleotide (50 µL, typically 300 µM) in 100 mM sodium phosphate buffer (pH 7.2, 350 µL). The coupling reaction was allowed to proceed at 25 C for 1 h. The reaction mixture was directly loaded onto a NAP-5 size exclusion column to remove organic solvent, salts, and excess small molecules and was further purified by analytical scale reverse-phase HPLC (8-30% MeCN/0.1 M TEAA gradient). The desired oligonucleotide products were characterized by MALDI-TOF mass spectrometry.

3' 4-Formylbenzoate-Linked DNA (This reagent was used to capture the products of the template 7). The 4-formylbenzoate linked 20-mer DNA was prepared following the protocol for bromoacetate-linked DNA using 4-formyl benzoic acid instead of bromoacetic acid.

3' Succinic Acid Monoester-Linked DNA (This reagent was used to capture the products of the templates **1-6**). Succinic anhydride (22 mg, 0.1 mmol) was activated with NHS (10 mg, 0.1 mmol) in DMF (200 µL) at 25 °C for 15 min. 100 µL of the mixture was added to the 3' amino modified template (50 µL, typically 300 µM) in 100 mM HEPES buffer (pH 8.5; 850 µL) and was incubated at 37 °C for 16 h. The reaction mixture was desalted by NAP-5 size exclusion column and further purified by analytical scale HPLC (8-30% MeCN/0.1 M TEAA gradient). The desired oligonucleotide products were characterized by MALDI-TOF mass spectrometry.

### II. Results and Conclusions

### (i) DNA-templated Transfomation from an Azide to an Amine

A variety of organic azides linked to the 5' termini of 30-mer DNA oligonucleotide templates were reacted with a triphenylphosphine conjugated to the 3' terminus of a complementary DNA 10-mer (see, **FIG. 5A****).** DNA-templated azide-to-amine functional group transformations were performed by mixing a 30-mer 5' azide-linked template (12 pmol) and 10-mer 3' triphenylphosphine-linked reagent (24 pmol) in a total volume of 200 µL of 100 mM CAPS buffer (pH 10) containing 500 mM NaCl at 25 °C for 16 h. For substrates **4** and 5, 1 M NaCl and the addition of 0.5 mM DTT to inhibit phosphine oxidation was found to increase yields. Representative reaction conditions included for **1-7,** 60 nM azide, 120 nM phosphine, 0.1 M CAPS pH 10, 0.5 M NaCl; for **8-11,** as above, except 0.1 M MES pH 6.0, 1 M NaCl; and for 12, as above, except 0.1 M MOPS pH 7.5, 1 M NaCl.

Unlike DNA-templated coupling reactions, the azide-to-amine transformations could not be monitored directly by denaturing polyacrylamide gel electrophoresis (PAGE) because the starting materials and products had similar molecular weights. To assay the progress of these reactions, the putative amine products were captured with 20-mer-linked carboxylic acids in the presence of a carbodiimide, or with 20-mer-linked aldehydes in the presence of NaBH₃CN. These secondary reagents or capture reagents displaced the 10-mer linked phosphine oxide and efficiently coupled with primary amines, but not with azides. The resulting amide or secondary amine products gained the molecular weight of the 20-mer and could easily be distinguished from starting azides by PAGE.

In order to capture amine products derived from substrates **1-6** a 20-mer 3' carboxylic acid-linked reagent (24 µmol) was added to the reaction mixture with EDC (30 mM) and sulfo-NHS (15 mM) in MES buffer (pH 6.5). In order to capture amine products derived from substrate **7**, the product was captured with a 20-mer 3' aldehyde-linked reagent in the presence of NaBH₃CN (3 mM) in MES buffer (pH 6.5). Following product capture, the DNA-linked species were precipitated with NaOAc (pH 5), ethanol, and glycogen.

The resulting pellets were dissolved in denaturing gel-loading buffer and were subjected to denaturing PAGE analysis. Unless specified, denaturing PAGE analysis was performed using 15% polyacrylamide gel (TBE-urea).

Reaction yields were quantitated by ethidium bromide staining of the gels, UV visualization and CCD-based densitometry of product and template bands. Yield calculations assumed that templates and products in denaturing gels stained with equal intensity per base. In cases where products were partially double-stranded during quantitation, changes in staining intensity may result in higher apparent yields.

Typical results obtained by denaturing PAGE analysis are shown in **FIG. 6. FIG. 6A** shows denaturing PAGE analysis of a DNA-templated azide-to-amine transformation for azide 3 in **FIG. 5B****.** **FIG. 6B** shows denaturing PAGE analysis of a DNA-templated azide-to-amine transformation for azide 7 in **FIG. 5B****.**

For the seven azides tested (substrates **1-7** in **FIG. 5B****)**, DNA-templated azide reduction proceeded efficiently at pH 10. The actual yields of the reaction products are summarized in **FIG. 5B****.** In each case, control reactions in which the phosphine was linked to a non-complementary, mismatched oligonucleotide did not generate significant amide or secondary amine products, indicating that these DNA-templated azide-to-amine transformations proceed sequence-specifically.

### (ii) DNA-templated Transformation from an Azide to Carboxylic Acid or Thiol

The scope of the reactions was further extended to effect azide-to carboxylic acid and azide-to-thiol functional group transformations (see, **FIG. 5A****).** In both cases, azide reduction induced spontaneous fragmentation to unmask carboxylic acid or thiol groups. To assess the efficiency of these reactions, DNA-linked amines were used to capture carboxylic acids (products resulting from substrates **8-10)** in the presence of a carbodiimide, while DNA-linked alkyl bromides were used to capture thiol products (products resulting from substrates **11** and **12).**

DNA-templated azide-to-carboxylic acid transformations were performed like the azide-to-amine transformations, except that the buffer contained 0.1 M MES pH 6.0 and 1 M NaCl. To capture carboxylic acid products, 20-mer 3' amine-linked reagent was added to the reaction mixture with EDC (30 mM) and sulfo-NHS (15 mM) in MES buffer (pH 6.5). Typical results from denaturing PAGE are shown in **FIG. 6C** (represented is the case using reagent **8** from **FIG. 5B****).**

DNA-templated azide-to-thiol transformations were performed as above, except that the buffer contained either 0.1 M MES pH 6.0 (for substrate **11)** or MOPS pH 7.5 (for substrate **12)** and 1 M NaCl. To capture thiol products, 20-mer 3' alkyl bromide-linked reagent was added to the reaction mixture and incubated at 37 °C for 6 h. Typical results from denaturing PAGE are shown in **FIG. 6D** (represented is the case using reagent **11** from **FIG. 5B****).**

For the carboxylic acid-to-amine and thiol-to-amine transformations, denaturing PAGE analysis indicated that DNA-templated functional group transformations to unmask carboxylic acid and thiol groups (substrates **8-12** in **FIG. 5B****)** also proceeded efficiently and sequence-specifically.

### Example 2. DNA-Templated Transformation of Azides into Primary Amines, Carboxylic Acids, and Thiols (Characterization by Mass Spectrometry)

This Example is similar to Example 1 except the reaction products were characterized by mass spectrometry rather than PAGE. To facilitate this a smaller template and different capture system were used under the same or similar conditions.

### I. Materials and Methods

### (i) Synthesis of Azido Acids

The azido substrates for the synthesis of compounds **1-12** shown in **FIG. 5B** were prepared as described in Example 1.

### (ii) Preparation of Functionalized Oligonucleotides

The oligonucleotides used in this Example were prepared in a manner similar to Example 1 with the following changes.

### (a) Template Oligonucleotides

The template oligonucleotides were prepared as described in Example 1 except that rather than using a 30-mer template, the following 10-mer template was used: 5'-NH₂(C₂H₄O)₂-PO₃H-GGT ACG AAT T-OPO₃H-CH(CH₂OH)CH₂(OC₂H₄)₄CH₂NHCO-biotin (SEQ ID NO: 5).

### (b) Transfer Units

The triphenylphosphine-linked reagent was prepared as described in Example 1.

### (iii) Mass Spectroscopic Analysis

MALDI-TOF mass spectrometry was performed on an Applied Biosystems Voyager-DE Pro Biospectrometry Workstation and processed with Voyager Data Explorer software. A mixture of nine parts hydroxypicolinic acid (HPA, 50 mg/mL in 50% MeCN/H₂O) and one part ammonium citrate (50 mg/mL in H₂O) was used as the matrix in all experiments.

### II. Results and Conclusions

Complementary DNA-linked phosphine reagent (24 pmol) was added to a solution of 10-mer 5'-azide-linked, 3'-biotinylated template (12 pmol) in 100 mM CAPS buffer (pH 10) with 500 mM NaCl. The mixture was agitated at 25° C for 0.5 h then at 37 °C for 12 h. The biotinylated products and unreacted templates were purified by treating the reaction mixture with streptavidin-linked magnetic particles (Roche) and eluted following the manufacturer's protocol. DNA in the eluant was precipitated with ethanol and glycogen. Substrates **11-12** were subjected directly to subsequent mass spectroscopic analysis. Samples for MALDI-TOF analysis were prepared by desalting the pellets dissolved in the matrix solution using a ZipTip C18 column (Millipore).

The resulting iminophosphoranes were identified by MALDI-TOF mass spectrometry and found to be unexpectedly stable to hydrolysis especially under acidic conditions, presumably due to formation of a stable HCl salt (Shalev, et al. (1996) J. ORG. CHEM. 61: 1689-1701). Treatment of template-linked azides with DNA-linked phosphine in pH 10 buffer at 25 °C for 0.5 h followed by 37 °C for 12 h, however, resulted in quantitative iminophosphorane hydrolysis to generate the corresponding primary amines.

The results from the MALDI-TOF analysis are summarized in Table 1 where reagents **1-12** are denoted as in **FIG. 5B****.** Due to the instability under the conditions for MALDI-TOF experiments, thiol-linked products **(11-12)** were captured as alkyl thioether adducts by treating with iodoacetamide (5 mM) following the DNA-templated Staudinger reaction (the MALDI-TOF data for **11-12** in Table 1 are of captured thioether adducts).

**Table 1**

| **Reagents** **(see,** **FIG. 5B****)** | Expected Mass | Observed Mass |
|---|---|---|
| **1** | 5866.05 | 5868.02 ± 9 |
| **2** | 5922.16 | 5926.50 ± 9 |
| **3** | 5942.15 | 5945.18 ± 9 |
| **4** | 5937.13 | 5940.98 ± 9 |
| **5** | 5934.17 | 5934.61 ± 9 |
| **6** | 5942.15 | 5944.46 ± 9 |
| **7** | 5928.12 | 5930.91 ± 9 |
| **8** | 5963.16 | 5968.42 ± 9 |
| **9** | 5909.07 | 5915.97 ± 9 |
| **10** | 6032.23 | 6038.24 ± 9 |
| **11** | 5941.16 | 5944.61 ± 9 |
| **12** | 5955.19 | 5957.01 ± 9 |

Mass spectrometric analysis of azide reduction reactions was consistent in each case with the formation of expected primary amine products. A representative spectrum is shown in **FIG. 7****.**

Based on the mass spectroscopic analyses set forth in Table 1, the sequence specific azide-to-amine, azide-to-carboxylic acid, and azide-to-thiol transformations all produced the appropriate products.

### Example 3. Transformations of Amine-Linked Templates Using Small Molecule Reagents

To further explore the ability of DNA-templated functional group transformations to enable non-DNA-linked reagents to participate in sequence-programmed synthesis, four DNA templates (templates **13-16,** **FIG. 8****)** were prepared, each containing a different azide at the 5' terminus, one of four unique six-base codons, and a biotin group at the 3' terminus to facilitate template manipulation and purification. The azide-linked templates then were chemically converted into amine-linked templates by exposure to TCEP-HCl. The resulting amines then were reacted with free reagents to determine whether the conversion of the amine intermediate into a final product was possible. In particular, dansyl chloride **(21),** ethyl chloroformate **(22),** 4-methoxy phenyl isocyanate **(23)** and 6-morpholino pyridinyl 3-isothiocyanate **(24)** were all chosen as amine-reactive agents as they cannot easily be attached to DNA due to their structure or their reactivity with water. Simplified reaction schemes showing the starting reagents and theoretical end products are shown in **FIG. 9****.**

### I. Materials and Methods

Coding sequences for the templates were designed by computational screening to (*i*) ensure that at least 6 non-complementary base pairs existed between any two different codons,

### (ii) maintain a constant %GC per codon in order to minimize differences in melting temperatures between reagents, and (iii) vary in mass such that the molecular weights of the 16 theoretical small-molecule coupling products are distinct and identifiable by MALDI-TOF mass spectrometry.

Each of the templates used in the schemes shown in **FIG. 8** (templates **13-16)** contained 5' NH₂(C₂H₄O)₂-PO₃H-TT-(codon)-GTAₙ-OPO₃H-CH(CH₂OH)CH₂(OC₂H₄)₄CH₂NHCO-biotin. The codons used for each template were as follows: the codon for template **13** was GTG CAA CGT CAT, n = 0 (SEQ ID NO: 6); the codon for template **14** was CCT AGT CGT CAT, n = 3 (SEQ ID NO: 7); the codon for template **15** was TAA GCC CGT CAT, n = 2 (SEQ ID NO: 8); and the codon for template **16:** AGC TTG CGT CAT, n = 1 (SEQ ID NO: 9).

The azide-containing templates **13-16,** were prepared as described in Example 1 (see templates 1, 3, 4, and 2, respectively).

The azide groups in templates **13-16** were chemically transformed into amine groups by exposure to TCEP-HCl. Briefly, amine-linked templates then were prepared by treating the azide-linked templates (templates **13-16)** with 5 mM TCEP-HCl in 100 mM MOPS buffer (pH 7.5) at 25 °C for 3 h. The resulting templates were purified by HPLC. Thereafter, the resulting templates (amine intermediates) were reacted with soluble reagents to determine whether functional group transformations were possible.

### II. Results and Conclusions

Once the amine-linked templates **13-16** were created, they were then exposed to soluble reagents to see whether functional group transformations were possible. Each of the transformations are discussed in detail below.

The amine-linked template **13** (400 pmol) in 100 µL of 100 mM aqueous NaHCO₃ (pH 9.0) was mixed with 20 mM dansyl chloride **21** in 100 µL DMF and agitated at 37 °C for 1 h. The reaction mixture was diluted in 200 µL 0.1 M TEAA and passed through a NAP-5 size exclusion column. The eluant in 1 mL 0.1 M TEAA was analyzed by analytical scale reverse phase HPLC (8-30 % MeCN/ 0.1 M TEAA gradient). Product yield was calculated based on the integrated peak areas (based on UV absorbance at 260 nm) of the starting material, the product, and any side products. A representative chromatogram is shown in **FIG. 10A****.**

Amine-linked template **14** (400 pmol) in 100 µL of 200 mM aqueous NaHCO₃ (pH 9.0) was mixed with 40 mM ethyl chloroformate **22** in 100 µL DMF and agitated at 37 °C for 1 h. The reaction was quenched by addition of glycogen in NaOAc buffer (pH 5.0) followed by ethanol precipitation. The pellet was dissolved in 0.1 M TEAA and analyzed by analytical scale reverse phase HPLC (8-30 % MeCN/ 0.1 M TEAA gradient). A representative chromatogram is shown in **FIG. 10B****.**

Amine-linked template **15** (400 pmol) in 100 µL of 500 mM aqueous triethylamine (pH 10) was mixed with 20 mM 4-methoxyphenylisocyanate **23** in 100 µL DMF and agitated at 37 °C for 1 h. The reaction mixture was quenched and analyzed as described above. A representative chromatogram is shown in **FIG. 10C****.**

Amine-linked template **16** (400 pmol) in 100 µL of 500 mM aqueous triethylamine (pH 10) was mixed with 20 mM and was allowed to react with 20 mM 6-morpholino isothiocyanate **24** in 100 µL DMF and agitated at 37°C for 1 h. The reaction mixture was quenched and analyzed as described above. A representative chromatogram is shown in **FIG. 10D****.**

When soluble reagents (free reactants) **21, 22, 23,** or **24** were added in excess (10 or 20 mM final concentration) in DMF to a template-linked primary amine under basic conditions (pH 9-10), the corresponding sulfonamide, carbamate, urea, or thiourea was efficiently generated (70% yield for **21,** > 86% for **22, 23** and **24).** These results demonstrated that it was possible to convert a template coupled to an amide into a sulfonamide, a carbomate, urea or thiourea using free reactants.

### Example 4. Sequence Specific Transformation of Four Azide-linked Templates Using Small Molecule Reagents

This Example demonstrates that it is possible to perform a sequence specific transformation of template bound reactants to generate reaction intermediates, which can then be reacted with free reactants to produce reaction products. In particular, a single-solution mixture of azide linked templates were sequence-specifically transformed into amine intermediates. The amine intermediates were then sequence-specifically modified into sulfonamide, carbamate, urea, and thiourea products using sulfonyl chloride, chloroformate, isocyanate, and isothiocyanate reactants not linked to DNA.

### I. Materials and Methods

### (i) Preparation of Functionalized Oligonucleotides

### (a) Template Oligonucleotides

The templates **13-16** were prepared as described in Example 3.

### (b) Transfer Units

Each of the following triphenylphosphine-linked oligonucleotides were prepared as described in Example 1.

Oligonucleotide **17 (****FIG. 9****)** had the structure 5' CGT TGC ACA A- OPO₃H-CH₂CH(CH₂OH)(CH₂)₄NHCOC₆H₄PPh₂ (SEQ ID NO: 10). Oligonucleotide **18 (****FIG. 9****)** had the structure 5' CGA CTA GGA A- OPO₃H-CH₂CH(CH₂OH)(CH₂)₄NHCOC₆H₄PPh₂ (SEQ ID NO: 11). Oligonucleotide **19 (****FIG. 9****)** had the structure 5' CGG GCT TAA A- OPO₃H-CH₂CH(CH₂OH)(CH₂)₄NHCOC₆H₄PPh₂ (SEQ ID NO: 12). Oligonucleotide **20 (****FIG. 9****)** had the structure 5' CGC AAG CTA A- OPO₃H-CH₂CH(CH₂OH)(CH₂)₄NHCOC₆H₄PPh₂ (SEQ ID NO: 13).

### II. Results and Conclusions

A mixture of templates **13-16** was combined with sequence specific reactant **17** and then free reactant **21.** The resulting solution was similarly combined with sequence specific reactant **18** followed by free reactant **22;** sequence specific reactant **19** followed by free reactant **23;** and sequence specific reactant **20** followed by free reactant **24.**

More specifically, 3' triphenylphosphine-linked oligonucleotide **17** (8 equiv.) was added to a single solution mixture of the four 5' azide-linked templates (templates **13-16,** 100 nM for each template) in 100 mM CAPS buffer (pH 10) and 500 mM NaCl to effect azide-to-amine transformation. The mixture was incubated at 25 °C for 0.5 h then 37 °C for 12 h. The oligonucleotides were precipitated by the addition of glycogen in NaOAc buffer (pH 5.0) and ethanol. The pellet was dissolved in 100 µL of 100 mM NaHCO₃ and was allowed to react with dansyl chloride **21** in 100 µL of DMF (20 mM) at 37 °C for 1 h. The reaction mixture was desalted by ethanol precipitation. If the DNA-templated azide-to-amine transformation proceeded sequence-specifically, only the amine arising from template **13** should react with sequence specific reactant **21** to generate sulfonamide **25,** while templates **14-16** should remain unaltered (see, **FIG. 8****).** Excess sulfonyl chloride was removed upon ethanol precipitation, and any unreacted amines were removed using N-hydroxysuccinimidyl (NHS) ester-linked resin.

The DNA-templated azide-to-amine transformation described above then was repeated using phosphine-linked oligonucleotide **18.** The pellet was dissolved in 100 µL of 200 mM NaHCO₃ and was allowed to react with ethyl chloroformate **22** in 100 µL of DMF (40 mM) at 37 °C for 1 h. The reaction mixture was desalted by ethanol precipitation and dried.

The DNA-templated azide-to-amine transformation described above then was repeated using phosphine-linked oligonucleotide **19.** The pellet was dissolved in 100 µL of 500 mM aqueous triethylamine solution and was allowed to react with 4-methoxyphenylisocyanate **23** in 100 µL of DMF (20 mM) at 37 °C for 1 h. The reaction mixture was desalted by ethanol precipitation and dried.

The DNA-templated azide-to-amine transformation described above then was repeated using phosphine-linked oligonucleotide **20.** The pellet was dissolved in 100 µL of 500 mM aqueous triethylamine solution and was allowed to react with 6-morpholino-3-pyridinylisothiocyanate **24** in 100 µL of DMF (20 mM) at 37 °C for 1 h. The reaction mixture was desalted by ethanol precipitation and dried. The pellet was dissolved in 100 mM MES buffer (pH 6), first treated with TCEP-HCl (5 mM) at 25 °C for 2 h then with NHS activated resin (Amersham Biosciences; 5 µL resin solution for 100 pmol template) for another 2 h. The resin was removed by filtration and washed three times with 0.1 M TEAA.

The final mixture of products was purified by capturing template-linked biotin groups with streptavidin linked to magnetic particles. The captured oligonucleotides were eluted from the particles following the manufacturer's protocol. The DNA in the eluant was precipitated with NaOAc (pH 5.0), glycogen, and ethanol. DNA recovery was determined spectrometrically by monitoring UV absorbance for the starting material pool and the final product pool at 260 nm. The concentration for a mixture containing equal amounts of products **25-28** (see, **FIG. 8****)** with a UV absorbance of 1.0 at 260 nm was estimated to be 5.5 µM. Samples for MALDI-TOF analysis were prepared as described in Example 2. **FIG. 11A** and **FIG. 11B** show representative spectra of starting materials (templates **13, 14, 15** and **16)** and products (products **25, 26, 27, 28),** respectively.

MALDI-TOF mass spectrometry revealed that the final product mixture contained predominantly the four sequence-programmed products (sulfonamide **25,** carbamate **26,** urea **27,** and thiourea **28).** None of the 12 possible undesired cross-products were observed.

UV spectrometry analysis indicated that the final product mixture was generated in 51% overall yield for the four consecutive DNA-templated reduction and small-molecule coupling sequences. These results establish that DNA-templated functional group transformations enable non-DNA-linked small molecules to participate in sequence-programmed reactions. The efficiency of this process also highlights the value of molecular biology-based purification and washing strategies made possible when performing organic synthesis on this minute (sub-nmol) scale.

Taken together, the DNA-templated functional group transformation described in this Example expands the synthetic capabilities of nucleic acid-templated synthesis by addressing the need for reagents to be tethered to oligonucleotides. When the linkage of reagents to oligonucleotides is not possible or is inconvenient, these transformations allow such reagents to nevertheless contribute to small molecule syntheses while preserving the correspondence between an oligonucleotide sequence and a product structure.

## Claims

1. A method of synthesizing a reaction product, the method comprising the steps of:
(a) providing a mixture comprising a first reactive unit and a second reactive unit under conditions to induce a reaction between the first and second reactive units to produce a reaction intermediate;
(b) providing an oligonucleotide comprising an identifying sequence covalently attached to the reaction intermediate; and
(c) combining the reaction intermediate, co-existing with at least one of the reactive units, with a free reactant selectively reactive with the reaction intermediate, thereby synthesizing a reaction product linked to the identifying sequence, wherein the free reactant is more reactive with the reaction intermediate than with the at least one of the reactive units from the mixture provided in step (a).

2. The method of claim 1, wherein the identifying sequence is attached to the first reactive unit prior to the reaction that produces the reaction intermediate and remains linked to the reaction intermediate.

3. The method of claim 2, wherein the second reactive unit is attached to an oligonucleotide sequence complementary to the identifying sequence.

4. The method of claim 3, wherein step (a) comprises hybridizing the identifying sequence with the sequence complementary to the identifying sequence, thereby bringing the first and second reactive units into reactive proximity.

5. The method of claim 1, wherein step (b) comprises enzymatically attaching the identifying sequence to the reaction intermediate after formation of the reaction intermediate.

6. A method of synthesizing a reaction product by nucleic acid-templated synthesis, the method comprising the steps of:
(a) providing a mixture comprising (i) a first reactive unit attached to a first oligonucleotide comprising a codon sequence, and (ii) a second reactive unit attached to a second oligonucleotide comprising an anti-codon sequence complementary to the codon sequence;
(b) annealing the codon sequence of the first oligonucleotide with the anti-codon sequence of the second oligonucleotide to induce a reaction between the first and second reactive units to form a reaction intermediate covalently attached at least to the first oligonucleotide; and
(c) combining the reaction intermediate co-existing with at least one of the reactive units with a free reactant selectively reactive with the reaction intermediate, thereby synthesizing a reaction product linked to the first oligonucleotide, wherein the free reactant is more reactive with the reaction intermediate than with the at least one of the reactive units from the mixture provided in step (a).

7. A method of synthesizing a reaction product, the method comprising the steps of:
(a) providing a mixture of a population of first reactive units and a second reactive unit under conditions that induce a reaction between at least one of the first reactive units and the second reactive unit, thereby to form a reaction intermediate co-existing with the remaining population of unreacted first reactive units;
(b) providing an oligonucleotide comprising an identifying sequence covalently attached to the reaction intermediate;
(c) combining the reaction intermediate co-existing with the first reactive units with a free reactant capable of selectively reacting with the reaction intermediate, thereby synthesizing a reaction product attached to an identifying sequence, the reaction product co-existing with the population of first reactive units, wherein the free reactant is more reactive with the reaction intermediate than with at least one of the reactive units in the starting mixture.

8. The method of claim 7, wherein the identifying sequence is attached to the at least one of the first reactive units prior to and during the reaction to produce the reaction intermediate and remains attached to the reaction intermediate.

9. The method of claim 8, wherein the second reactive unit is linked to a sequence complementary to the identifying sequence.

10. The method of claim 9, wherein step (a) comprises hybridizing the identifying sequence with the sequence complementary to the identifying sequence, thereby bringing the at least one of the first reactive units and the second reactive unit into reactive proximity.

11. The method of claim 7, wherein step (b) comprises enzymatically attaching the identifying sequence to the reaction intermediate after formation of the reaction intermediate.

12. The method of any preceding claim wherein the reaction product is synthesized with a yield greater than or equal to 50%, greater than or equal to 75%, greater than or equal to 85%, or greater than or equal to 98%.

13. A method of synthesizing a reaction product by nucleic acid-templated synthesis, the method comprising the steps of:
(a) providing a mixture comprising a plurality of different first reactive units each attached to corresponding different first oligonucleotides comprising a codon sequence, wherein the oligonucleotide sequence is indicative of the first reactive unit attached thereto;
(b) providing a second reactive unit attached to a second oligonucleotide comprising an anti-codon sequence complementary to the codon sequence of at least one first reactive unit;
(c) annealing the codon sequence of at least one of the first oligonucleotides with the anti-codon sequence of the second oligonucleotide to induce a reaction between the first and second reactive units to form a first reaction intermediate covalently attached at least to a first oligonucleotide; and
(d) combining the first reaction intermediate, co-existing with at least one of the reactive units, with a free reactant selectively reactive with the first reaction intermediate, thereby synthesizing a first reaction product attached to the first oligonucleotide, wherein the free reactant is more reactive with the first reaction intermediate than with the at least one of the reactive units from the mixture.

14. The method of claim 13, further comprising the steps of:
(e) providing a third reactive unit attached to a third oligonucleotide comprising an anti-codon sequence complementary to the codon sequence of at least one first reactive unit;
(f) annealing the codon sequence of at least one of the first oligonucleotides with the anti-codon sequence of the third oligonucleotide to induce a reaction between the first and third reactive units to form a second reaction intermediate attached to the at least one of the first oligonucleotides; and
(g) combining the second reaction intermediate with a free reactant selectively reactive with the second reaction intermediate, thereby synthesizing a second reaction product attached to the at least one of the first oligonucleotides, wherein the free reactant is more reactive with the second reaction intermediate than with at least one of the reactive units in the mixture.

15. An *in vitro* method of performing nucleic acid-templated synthesis, the method comprising the steps of:
(a) providing a mixture comprising (i) a plurality of different templates each comprising a first reactive unit covalently attached to a first oligonucleotide defining a codon sequence, and (ii) a transfer unit comprising a second reactive unit covalently attached to a second oligonucleotide defining an anti-codon sequence complementary to the codon sequence of the template;
(b) annealing the codon sequence of one template with the anti-codon sequence of the transfer unit to bring the first reactive unit and the second reactive unit into reactive proximity so that the first and second reactive units react with one another to produce a reaction intermediate; and
(c) contacting the reaction intermediate, while co-existing with unreacted template, with a free reactant, which chemically reacts with the reaction intermediate to produce a reaction product, wherein the free reactant is more reactive with the reaction intermediate than with at least one of the reactive units in the starting mixture and wherein the first oligonucleotide remains attached to the reaction product.

16. The method of claim 15, wherein the first reactive unit is a small molecule scaffold.

17. The method of claim 15, wherein, in step (b), a functional group of the first reactive unit is transformed into a different chemical moiety in the reaction intermediate.

18. The method of claim 16, wherein the small molecule scaffold contains a protected functional group.

19. The method of claim 18, wherein, in step (b), the functional group is deprotected to produce a reaction intermediate where the small molecule scaffold contains a deprotected functional group.

20. The method of claim 16, wherein the reaction product
(a) is not a nucleic acid; or
(b) is not a nucleotide or a nucleotide analog; or
(c) is not a nucleotide analog.

21. The method of claim 15, wherein, in step (b), the first and second reactive units react with one another to produce the reaction intermediate without the assistance of a ribosome.

22. The method of claim 15 further comprising the step of selecting the reaction product attached to the first oligonucleotide.

23. The method of claim 22 further comprising the step of amplifying the first oligonucleotide.

24. The method of claim 23 comprising the additional step of determining the sequence of the first oligonucleotide attached to the reaction product so as to determine the identity or synthetic history of the reaction product.

25. The method of any preceding claim, wherein the free reactant is at least five times more reactive, or at least fifty times more reactive, or at least one thousand times more reactive, with the reaction intermediate than with all of, or at least one of, the reactive units in the starting mixture.

## Patentansprüche

1. Verfahren zur Synthese eines Reaktionsprodukts, wobei das Verfahren die folgenden Schritte umfasst:
(a) Bereitstellen eines Ansatzes mit einer ersten reaktiven Einheit und einer zweiten reaktiven Einheit unter Bedingungen, so dass eine Reaktion zwischen der ersten und der zweiten reaktiven Einheit unter Erhalt einer Reaktionszwischenstufe induziert wird;
(b) Bereitstellen eines Oligonukleotids mit einer kovalent an die Reaktionszwischenstufe gebundenen Identifizierungssequenz; und
(c) Kombinieren der Reaktionszwischenstufe, die mit wenigstens einer der reaktiven Einheiten zusammen vorliegt, mit einem mit der Reaktionszwischenstufe selektiv reaktiven freien Reaktanden, wodurch ein mit der Identifizierungssequenz verknüpftes Reaktionsprodukt synthetisiert wird, wobei der freie Reaktand eine stärkere Reaktivität mit der Reaktionszwischenstufe als mit der wenigstens einen der reaktiven Einheiten aus dem in Schritt (a) bereitgestellten Ansatz aufweist.

2. Verfahren nach Anspruch 1, wobei die Identifizierungssequenz vor der Reaktion, bei der die Reaktionszwischenstufe erhalten wird, an die erste reaktive Einheit gebunden wird und mit der Reaktionszwischenstufe verknüpft bleibt.

3. Verfahren nach Anspruch 2, wobei die zweite reaktive Einheit an eine zur Identifizierungssequenz komplementäre Oligonukleotidsequenz gebunden wird.

4. Verfahren nach Anspruch 3, wobei Schritt (a) das Hybridisieren der Identifizierungssequenz mit der zur Identifizierungssequenz komplementären Sequenz umfasst, wodurch die erste und die zweite reaktive Einheit in reaktive Nähe zueinander gebracht werden.

5. Verfahren nach Anspruch 1, wobei Schritt (b) das enzymatische Binden der Identifizierungssequenz an die Reaktionszwischenstufe nach Bildung der Reaktionszwischenstufe umfasst.

6. Verfahren zur Synthese eines Reaktionsprodukts mittels nukleinsäurematrizengesteuerter Synthese, wobei das Verfahren die folgenden Schritte umfasst:
(a) Bereitstellen eines Ansatzes mit (i) einer an ein erstes Oligonukleotid mit einer Codonsequenz gebundenen ersten reaktiven Einheit und (ii) einer an ein zweites Oligonukleotid mit einer zu der Codonsequenz komplementären Anti-Codonsequenz gebundenen zweiten reaktiven Einheit;
(b) Annealing der Codonsequenz des ersten Oligonukleotids mit der Anti-Codonsequenz des zweiten Oligonukleotids, so dass eine Reaktion zwischen der ersten und der zweiten reaktiven Einheit unter Bildung einer wenigstens an das erste Oligonukleotid kovalent gebundenen Reaktionszwischenstufe induziert wird; und
(c) Kombinieren der mit wenigstens einer der reaktiven Einheiten zusammen vorliegenden Reaktionszwischenstufe mit einem mit der Reaktionszwischenstufe selektiv reaktiven freien Reaktanden, wodurch ein mit dem ersten Oligonukleotid verknüpftes Reaktionsprodukt synthetisiert wird, wobei der freie Reaktand eine stärkere Reaktivität mit der Reaktionszwischenstufe als mit der wenigstens einen der reaktiven Einheiten aus dem in Schritt (a) bereitgestellten Ansatz aufweist.

7. Verfahren zur Synthese eines Reaktionsprodukts, wobei das Verfahren die folgenden Schritte umfasst:
(a) Bereitstellen eines Ansatzes einer Population erster reaktiver Einheiten und einer zweiten reaktiven Einheit unter Bedingungen, durch die eine Reaktion zwischen wenigstens einer der ersten reaktiven Einheiten und der zweiten reaktiven Einheit unter Erhalt einer Reaktionszwischenstufe induziert wird, um dadurch eine mit der verbliebenen Population nicht umgesetzter erster reaktiver Einheiten zusammen vorliegende Reaktionszwischenstufe zu bilden;
(b) Bereitstellen eines Oligonukleotids mit einer kovalent an die Reaktionszwischenstufe gebundenen Identifizierungssequenz;
(c) Kombinieren der mit den ersten reaktiven Einheiten zusammen vorliegenden Reaktionszwischenstufe mit einem zur selektiven Reaktion mit der Reaktionszwischenstufe fähigen freien Reaktanden, wodurch ein an eine Identifizierungssequenz gebundenes Reaktionsprodukt synthetisiert wird, wobei das Reaktionsprodukt mit der Population erster reaktiver Einheiten zusammen vorliegt, wobei der freie Reaktand eine stärkere Reaktivität mit der Reaktionszwischenstufe als mit wenigstens einer der reaktiven Einheiten im Ausgangsansatz aufweist.

8. Verfahren nach Anspruch 7, wobei die Identifizierungssequenz vor und während der Reaktion zur Erzeugung der Reaktionszwischenstufe an die wenigstens eine der ersten reaktiven Einheiten gebunden wird und an die Reaktionszwischenstufe gebunden bleibt.

9. Verfahren nach Anspruch 8, wobei die zweite reaktive Einheit mit einer zur Identifizierungssequenz komplementären Sequenz verknüpft wird.

10. Verfahren nach Anspruch 9, wobei Schritt (a) das Hybridisieren der Identifizierungssequenz mit der zur Identifizierungssequenz komplementären Sequenz umfasst, wodurch die wenigstens eine der ersten reaktiven Einheiten und die zweite reaktive Einheit in reaktive Nähe zueinander gebracht werden.

11. Verfahren nach Anspruch 7, wobei Schritt (b) das enzymatische Binden der Identifizierungssequenz an die Reaktionszwischenstufe nach Bildung der Reaktionszwischenstufe umfasst.

12. Verfahren nach einem der vorhergehenden Ansprüche, wobei das Reaktionsprodukt mit einer Ausbeute von größer oder gleich 50%, größer oder gleich 75%, größer oder gleich 85% oder größer oder gleich 98% synthetisiert wird.

13. Verfahren zur Synthese eines Reaktionsprodukts mittels nukleinsäurematrizengesteuerter Synthese, wobei das Verfahren die folgenden Schritte umfasst:
(a) Bereitstellen eines Ansatzes mit mehreren unterschiedlichen ersten reaktiven Einheiten, die jeweils an entsprechende erste Oligonukleotide mit einer Codonsequenz gebunden sind, wobei die Oligonukleotidsequenz die daran gebundene erste reaktive Einheit anzeigt;
(b) Bereitstellen einer an ein zweites Oligonukleotid mit einer zu der Codonsequenz wenigstens einer ersten reaktiven Einheit komplementären Anti-Codonsequenz gebundenen zweiten reaktiven Einheit;
(c) Annealing der Codonsequenz wenigstens eines der ersten Oligonukleotide mit der Anti-Codonsequenz des zweiten Oligonukleotids, so dass eine Reaktion zwischen der ersten und der zweiten reaktiven Einheit unter Bildung einer wenigstens an ein erstes Oligonukleotid kovalent gebundenen ersten Reaktionszwischenstufe induziert wird; und
(d) Kombinieren der ersten Reaktionszwischenstufe, die mit wenigstens einer der reaktiven Einheiten zusammen vorliegt, mit einem mit der ersten Reaktionszwischenstufe selektiv reaktiven freien Reaktanden, wodurch ein an das erste Oligonukleotid gebundenes erstes Reaktionsprodukt synthetisiert wird, wobei der freie Reaktand eine stärkere Reaktivität mit der ersten Reaktionszwischenstufe als mit der wenigstens einen der reaktiven Einheiten aus dem Ansatz aufweist.

14. Verfahren nach Anspruch 13, das ferner die folgenden Schritte umfasst:
(e) Bereitstellen einer an ein drittes Oligonukleotid mit einer zu der Codonsequenz wenigstens einer ersten reaktiven Einheit komplementären Anti-Codonsequenz gebundenen dritten reaktiven Einheit;
(f) Annealing der Codonsequenz wenigstens eines der ersten Oligonukleotide mit der Anti-Codonsequenz des dritten Oligonukleotids, so dass eine Reaktion zwischen der ersten und der dritten reaktiven Einheit unter Bildung einer an das wenigstens eine der ersten Oligonukleotide gebundenen zweiten Reaktionszwischenstufe induziert wird; und
(g) Kombinieren der zweiten Reaktionszwischenstufe mit einem mit der zweiten Reaktionszwischenstufe selektiv reaktiven freien Reaktanden, wodurch ein an das wenigstens eine der ersten Oligonukleotide gebundenes zweites Reaktionsprodukt synthetisiert wird, wobei der freie Reaktand eine stärkere Reaktivität mit der zweiten Reaktionszwischenstufe als mit wenigstens einer der reaktiven Einheiten in dem Ansatz aufweist.

15. In-vitro-Verfahren zur Durchführung von nukleinsäurematrizengesteuerter Synthese, wobei das Verfahren die folgenden Schritte umfasst:
(a) Bereitstellen eines Ansatzes mit (i) mehreren unterschiedlichen Matrizen, die jeweils eine an ein eine Codonsequenz definierendes erstes Oligonukleotid kovalent gebundene erste reaktive Einheit umfassen, und (ii) einer Transfer-Einheit, die eine an ein eine zur Codonsequenz der Matrize komplementäre Anti-Codonsequenz definierendes zweites Oligonukleotid kovalent gebundene zweite reaktive Einheit umfasst;
(b) Annealing der Codonsequenz einer Matrize mit der Anti-Codonsequenz der Transfer-Einheit, um die erste reaktive Einheit und die zweite reaktive Einheit in reaktive Nähe zueinander zu bringen, so dass die erste und die zweite reaktive Einheit unter Bildung einer Reaktionszwischenstufe miteinander reagieren; und
(c) Inkontaktbringen der Reaktionszwischenstufe, während diese mit nicht umgesetzter Matrize zusammen vorliegt, mit einem freien Reaktanden, der eine chemische Reaktion mit der Reaktionszwischenstufe unter Erhalt eines Reaktionsprodukts eingeht, wobei der freie Reaktand eine stärkere Reaktivität mit der Reaktionszwischenstufe als mit wenigstens einer der reaktiven Einheiten im Ausgangsansatz aufweist und wobei das erste Oligonukleotid an das Reaktionsprodukt gebunden bleibt.

16. Verfahren nach Anspruch 15, wobei es sich bei der ersten reaktiven Einheit um ein kleines Molekülgerüst handelt.

17. Verfahren nach Anspruch 15, wobei in Schritt (b) eine funktionelle Gruppe der ersten reaktiven Einheit in eine andere chemische Gruppierung in der Reaktionszwischenstufe umgewandelt wird.

18. Verfahren nach Anspruch 16, wobei das kleine Molekülgerüst eine geschützte funktionelle Gruppe enthält.

19. Verfahren nach Anspruch 18, wobei in Schritt (b) die funktionelle Gruppe unter Erhalt einer Reaktionszwischenstufe entschützt wird und das kleine Molekülgerüst eine entschützte funktionelle Gruppe enthält.

20. Verfahren nach Anspruch 16, wobei es sich bei dem Reaktionsprodukt
(a) nicht um eine Nukleinsäure handelt; oder
(b) weder um ein Nukleotid noch ein Nukleotidanalog handelt; oder
(c) nicht um ein Nukleotidanalog handelt.

21. Verfahren nach Anspruch 15, wobei in Schritt (b) die erste und die zweite reaktive Einheit miteinander unter Erhalt der Reaktionszwischenstufe ohne Hilfe eines Ribosoms reagieren.

22. Verfahren nach Anspruch 15, das ferner den Schritt des Auswählens des an das erste Oligonukleotid gebundenen Reaktionsprodukts umfasst.

23. Verfahren nach Anspruch 22, das ferner den Schritt des Amplifizierens des ersten Oligonukleotids umfasst.

24. Verfahren nach Anspruch 23, das den zusätzlichen Schritt des Bestimmens der Sequenz des an das Reaktionsprodukt gebundenen ersten Oligonukleotids umfasst, um die Identität oder die Synthesegeschichte des Reaktionsprodukts zu bestimmen.

25. Verfahren nach einem der vorhergehenden Ansprüche, wobei der freie Reaktand eine wenigstens fünfmal höhere oder wenigstens fünfzigmal höhere oder wenigstens tausendmal höhere Reaktivität mit der Reaktionszwischenstufe als mit allen oder mit wenigstens einer der reaktiven Einheiten im Ausgangsansatz aufweist.

## Revendications

1. Procédé de synthèse d'un produit de réaction, le procédé comprenant les étapes consistant à :
(a) produire un mélange comprenant une première unité réactive et une deuxième unité réactive dans des conditions pour induire une réaction entre les première et deuxième unités réactives pour produire un intermédiaire de réaction ;
(b) produire un oligonucléotide comprenant une séquence d'identification liée de manière covalente à l'intermédiaire de réaction ; et
(c) combiner l'intermédiaire de réaction, coexistant avec au moins une des unités réactives, avec un réactif libre sélectivement réactif avec l'intermédiaire de réaction, de manière à synthétiser un produit de réaction lié à la séquence d'identification, **caractérisé en ce que** le réactif libre est plus réactif avec l'intermédiaire de réaction qu'avec l'au moins une des unités réactives du mélange produit dans l'étape (a).

2. Procédé de la revendication 1, **caractérisé en ce que** la séquence d'identification est liée à la première unité réactive avant la réaction qui produit l'intermédiaire de réaction et reste liée à l'intermédiaire de réaction.

3. Procédé de la revendication 2, **caractérisé en ce que** la deuxième unité réactive est liée à une séquence oligonucléotidique complémentaire de la séquence d'identification.

4. Procédé de la revendication 3, **caractérisé en ce que** l'étape (a) comprend l'hybridation de la séquence d'identification avec la séquence complémentaire de la séquence d'identification, de manière à amener les première et deuxième unités réactives à proximité étroite.

5. Procédé de la revendication 1, **caractérisé en ce que** l'étape (b) comprend la liaison enzymatique de la séquence d'identification à l'intermédiaire de réaction après formation de l'intermédiaire de réaction.

6. Procédé de synthèse d'un produit de réaction par synthèse à partir de matrice d'acide nucléique, le procédé comprenant les étapes consistant à :
(a) produire un mélange comprenant (i) une première unité réactive liée à un premier oligonucléotide comprenant une séquence de codons, et (ii) une deuxième unité réactive liée à un deuxième oligonucléotide comprenant une séquence d'anticodons complémentaire de la séquence de codons ;
(b) hybrider la séquence de codons du premier oligonucléotide avec la séquence d'anticodons du deuxième oligonucléotide pour induire une réaction entre les première et deuxième unités réactives pour former un intermédiaire de réaction lié de manière covalente au moins au premier oligonucléotide ; et
(c) combiner l'intermédiaire de réaction coexistant avec au moins une des unités réactives avec un réactif libre sélectivement réactif avec l'intermédiaire de réaction, de manière à synthétiser un produit de réaction lié au premier oligonucléotide, **caractérisé en ce que** le réactif libre est plus réactif avec l'intermédiaire de réaction qu'avec l'au moins une des unités réactives du mélange produit dans l'étape (a).

7. Procédé de synthèse d'un produit de réaction, le procédé comprenant les étapes consistant à :
(a) produire un mélange d'une population de premières unités réactives et d'une deuxième unité réactive dans des conditions qui induisent une réaction entre au moins une des premières unités réactives et la deuxième unité réactive de manière à former un intermédiaire de réaction coexistant avec la population restante d'unités réactives n'ayant pas réagi ;
(b) produire un oligonucléotide comprenant une séquence d'identification liée de manière covalente à l'intermédiaire de réaction ;
(c) combiner l'intermédiaire de réaction coexistant avec les premières unités réactives avec un réactif libre capable de réagir sélectivement avec l'intermédiaire de réaction, de manière à synthétiser un produit de réaction lié à une séquence d'identification, le produit de réaction coexistant avec la population de premières unités réactives, **caractérisé en ce que** le réactif libre est plus réactif avec l'intermédiaire de réaction qu'avec l'au moins une des unités réactives dans le mélange de départ.

8. Procédé de la revendication 7, **caractérisé en ce que** la séquence d'identification est liée à l'au moins une des premières unités réactives avant et pendant la réaction pour produire l'intermédiaire de réaction et reste liée à l'intermédiaire de réaction.

9. Procédé de la revendication 8, **caractérisé en ce que** la deuxième unité réactive est liée à une séquence complémentaire de la séquence d'identification.

10. Procédé de la revendication 9, **caractérisé en ce que** l'étape (a) comprend l'hybridation de la séquence d'identification avec la séquence complémentaire de la séquence d'identification, de manière à amener les première unités réactives et la deuxième unité réactive à proximité réactive.

11. Procédé de la revendication 7, **caractérisé en ce que** l'étape (b) comprend la liaison enzymatique de la séquence d'identification à l'intermédiaire de réaction après la formation de l'intermédiaire de réaction.

12. Procédé de l'une quelconque des revendications précédentes **caractérisé en ce que** le produit de réaction est synthétisé avec un rendement supérieur ou égal à 50 %, supérieur ou égal à 75 %, supérieur ou égal à 85 %, ou supérieur ou égal à 98 %.

13. Procédé de synthèse d'un produit de réaction par synthèse à partir de matrice d'acide nucléique, le procédé comprenant les étapes consistant à :
(a) produire un mélange comprenant une pluralité d'unités réactives différentes, chacune liée à des premiers oligonucléotides différents correspondants comprenant une séquence de codons, où la séquence oligonucléotidique est indicative de la première unité réactive liée à celle-ci ;
(b) produire une deuxième unité réactive liée à un deuxième oligonucléotide comprenant une séquence d'anticodons complémentaire de la séquence de codons d'au moins une première unité réactive ;
(c) hybrider la séquence de codons d'au moins un des premiers oligonucléotides avec la séquence d'anticodons du deuxième oligonucléotide pour induire une réaction entre les premières et deuxième unités réactives pour former un premier intermédiaire de réaction lié de manière covalente au moins à un premier oligonucléotide ; et
(d) combiner le premier intermédiaire de réaction, coexistant avec au moins une des unités réactives, avec un réactif libre sélectivement réactif avec le premier intermédiaire de réaction, de manière à synthétiser un premier produit de réaction lié au premier oligonucléotide, **caractérisé en ce que** le réactif libre est plus réactif avec le premier intermédiaire de réaction qu'avec l'au moins une des unités réactives du mélange.

14. Procédé de la revendication 13, comprenant en outre les étapes consistant à :
(e) produire une troisième unité réactive liée à un troisième oligonucléotide comprenant une séquence d'anticodons complémentaire de la séquence de codons d'au moins une première unité réactive ;
(f) hybrider la séquence de codons d'au moins un des premiers oligonucléotides avec la séquence d'anticodons du troisième oligonucléotide pour induire une réaction entre les première et troisième unités réactives pour former un deuxième intermédiaire de réaction lié à l'au moins un des premiers oligonucléotides ; et
(g) combiner le deuxième intermédiaire de réaction avec un réactif libre sélectivement réactif avec le deuxième intermédiaire de réaction, de manière à synthétiser un deuxième produit de réaction lié à l'au moins un des premiers oligonucléotides, **caractérisé en ce que** le réactif libre est plus réactif avec le deuxième intermédiaire de réaction qu'avec au moins une des unités réactives dans le mélange.

15. Procédé *in vitro* pour effectuer la synthèse à partir de matrice d'acide nucléique, le procédé comprenant les étapes consistant à :
(a) produire un mélange comprenant (i) une pluralité de matrices différentes comprenant chacune une première unité réactive liée de manière covalente à un premier oligonucléotide définissant une séquence de codons, et (ii) une unité de transfert comprenant une deuxième unité réactive liée de manière covalente à un deuxième oligonucléotide définissant une séquence d'anticodons complémentaire de la séquence de codons de la matrice ;
(b) hybrider la séquence de codons d'une matrice avec la séquence d'anticodons de l'unité de transfert pour amener la première unité réactive et la seconde unité réactive à proximité réactive de sorte que les première et deuxième unités réactives réagissent l'une avec l'autre pour produire un intermédiaire de réaction ; et
(c) mettre en contact l'intermédiaire de réaction, coexistant avec la matrice n'ayant pas réagi, avec un réactif libre, qui réagit chimiquement avec l'intermédiaire de réaction pour produire un produit de réaction, **caractérisé en ce que** le réactif libre est plus réactif avec l'intermédiaire de réaction qu'avec au moins une des unités réactives dans le mélange de départ et **caractérisé en ce que** le premier oligonucléotide reste lié au produit de réaction.

16. Procédé de la revendication 15, **caractérisé en ce que** la première unité réactive est un échafaudage à petite molécule.

17. Procédé de la revendication 15, **caractérisé en ce que**, dans l'étape (b), un groupe fonctionnel de la première unité réactive est transformé en un fragment chimique différent dans l'intermédiaire de réaction.

18. Procédé de la revendication 16, **caractérisé en ce que** l'échafaudage à petite molécule contient un groupe fonctionnel protégé.

19. Procédé de la revendication 18, **caractérisé en ce que**, dans l'étape (b), le groupe fonctionnel est déprotégé pour produire un intermédiaire de réaction dans lequel l'échafaudage à petite molécule contient un groupe fonctionnel déprotégé.

20. Procédé de la revendication 16, **caractérisé en ce que** le produit de réaction
(a) n'est pas un acide nucléique ; ou
(b) n'est pas un nucléotide ou un analogue de nucléotide ; ou
(c) n'est pas un analogue de nucléotide.

21. Procédé de la revendication 15, **caractérisé en ce que**, dans l'étape (b), les première et deuxième unités réactives réagissent l'une avec l'autre pour produire l'intermédiaire de réaction sans l'aide d'un ribosome.

22. Procédé de la revendication 15 comprenant en outre l'étape de sélection du produit de réaction lié au premier oligonucléotide.

23. Procédé de la revendication 22 comprenant en outre l'étape d'amplification du premier oligonucléotide.

24. Procédé de la revendication 23 comprenant l'étape supplémentaire de détermination de la séquence du premier oligonucléotide lié au produit de réaction de manière à déterminer l'identité ou l'historique de synthèse du produit de réaction.

25. Procédé de l'une quelconque des revendications précédentes, **caractérisé en ce que** le réactif libre est au moins cinq fois plus réactif, ou au moins cinquante fois plus réactif, ou au moins mille fois plus réactif, avec l'intermédiaire de réaction qu'avec toutes, ou au moins une des unités réactives dans le mélange de départ.
